(19) Europäisches Patentamt
European Patent Office
Office européen des brevets

(11) Publication number: 0 181 129 B1

(12) EUROPEAN PATENT SPECIFICATION

(45) Date of publication of patent specification: 08.03.89

(51) Int. Cl.$^4$: C 07 H 19/167, A 61 K 31/70

(21) Application number: 85307717.0

(22) Date of filing: 25.10.85

# (54) N6-substituted deoxyribose analogs of adenosines.

(30) Priority: 26.10.84 US 665217
26.10.84 US 665232
26.10.84 US 665233
06.09.85 US 772315

(43) Date of publication of application:
14.05.86 Bulletin 86/20

(45) Publication of the grant of the patent:
08.03.89 Bulletin 89/10

(84) Designated Contracting States:
AT BE CH DE FR GB IT LI LU NL SE

(56) References cited:
DE-A-1 795 695
DE-A-2 402 804
US-A-3 475 408
US-A-4 373 097

CHEMICAL ABSTRACTS, vol. 82, 1975, page 483, abstract no. 73407r, Columbus, Ohio, US; & JP-A-74 17 278 (KOWA CO., LTD) 27-04-1974

(73) Proprietor: WARNER-LAMBERT COMPANY
201 Tabor Road
Morris Plains New Jersey 07950 (US)

(72) Inventor: Hamilton, Harriet W.
234 Park Street
Chelsea Michigan 48118 (US)
Inventor: Bristol, James A.
1921 High Hollow Drive
Ann Arbor Michigan 48103 (US)
Inventor: Moos, Walter
3760 Greenbrier Apt. 349B
Ann Arbor Michigan 48105 (US)
Inventor: Trivedi, Bharat K.
44833 Tillotson Drive
Canton Michigan 48187 (US)
Inventor: Taylor, Michael
3629 Partridge Path Apt. 6
Ann Arbor Michigan 48104 (US)
Inventor: Patt, William C.
523 Wilkinson
Chelsea Michigan 48118 (US)

(74) Representative: Jones, Michael Raymond et al
HASELTINE LAKE & CO. Hazlitt House
28 Southampton Buildings Chancery Lane
London WC2A 1AT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Dutch patent application 67:14189 describes $N^6$-cycloalkyl adenosines as cardioactive compounds. US Patent 3,851,056 discloses adenosines having an $N^6$-substituent of the formula

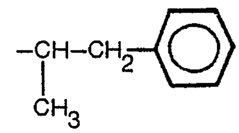

as having antilipolytic and antihyperlipaemic properties.

5'-Deoxy-5'-mercapto-$N^6$-(lower alkyl or benzyl) adenosines are described as antiviral agents in US patent 3,575,959. US Patent 4,373,097 describes compounds of the formula

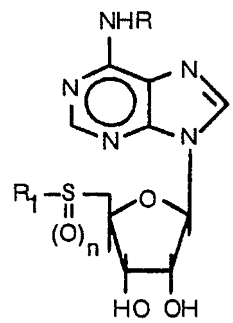

wherein n is 0, 1, or 2, $R^1$ is alkyl and R is hydrogen, aliphatic or aromatic acyl. These compounds are disclosed as having antiinflammatory and analgesic activity.

5'-Deoxy-5'-alkylthio-$N^6$-(saturated or unsaturated, straight or branched aliphatic hydrocarbon)adenosines having blood pressure reducing and blood vessel dilating activity are described in US patent 3,475,408.

5'-Deoxyribose derivatives of the formula

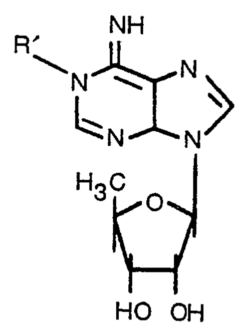

have been described in Japanese patent publication Number 55 8162—598 for treating thrombosis and embolisms.

$N^6$-Bicyclo[2.2.1]heptyladenosines having a 5' modified ribose having analgesic and antiinflammatory properties is disclosed in US Serial Number 665,216 now having a CIP copending with this application. Finally, $N^6$-(1- and 2-benzocycloalkyl)adenosines are disclosed in US 4,501,735.

The present compounds describe $N^6$-substituted deoxyribose analogs of adenosines as having analgesic, neuroleptic, and antihypertensive activity. The analogs include 5'-deoxy, 5'-deoxy-5'methylthio, and 5'-deoxy-5'-haloadenosines.

Accordingly, the present invention relates to a compound of the formula (I)

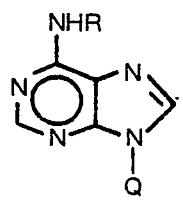

(I)

wherein R is cycloalkyl having a three to an eleven membered ring, or a group of the formula

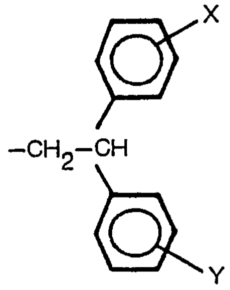 , 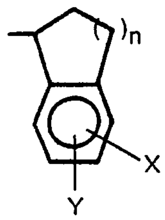 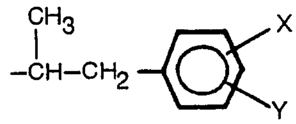 , or 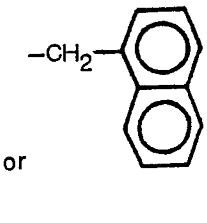

(Ia) (Ib) (Ic) (Ie)

in which n is one, two, or three; X and Y are each independently H, lower alkyl, hydroxy, lower alkoxy, benzyloxy, nitro, amino, trifluoromethyl, or halogen; Q is the formula

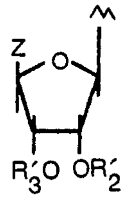

wherein Z is $CH_3$, $CH_2Hal$, or $CH_2SCH_3$; $R_2'$ or $R_3'$ are each independently H, lower alkyl, lower alkanoyl, benzoyl, benzoyl substituted by lower alkyl, lower alkoxy, or halogen, or when taken together are lower alkylidene, such as isopropylidene; or lower alkanoyl or benzoyl esters thereof; its diastereomers or mixtures thereof, or a pharmaceutically acceptable acid addition salt thereof.

The present invention also relates to a pharmaceutical composition comprising a therapeutically effective amount of a compound of the above Formula I with a pharmaceutically acceptable carrier, and to dosage forms of a compound of the Formula I as defined above.

In the compounds of the Formula I, the term "lower alkyl" is meant to include a straight or branched alkyl group having from 1 to 6 carbon atoms such as, for example, methyl, ethyl, propyl, isopropyl, butyl, *sec*-butyl, isobutyl, *tert*-butyl, amyl, isoamyl, neopentyl, hexyl, and the like.

Hal or halogen includes particularly fluorine, chlorine or bromine.

Lower alkoxy is O-alkyl of from one to six carbon atoms as defined above for "lower alkyl".

Lower alkanoyloxy is a stright or branched

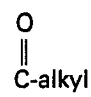

group of from 1 to 6 carbon atoms in the alkyl chain as defined above.

The compounds of Formula I are useful both in the free base form and in the form of acid addition salts. Both forms are within the scope of the invention. In practice, use of the salt form amounts to use of the base form. Appropriate pharmaceutically acceptable salts within the scope of the invention are those derived from mineral acids such as hydrochloric acid and sulfuric acid; and organic acids such as ethanesulfonic acid, benzenesulfonic acid, p-toluenesulfonic acid, and the like, giving the hydrochloride, sulfamate, ethanesulfonate, benzenesulfonate, p-toluenesulfonate, and the like, respectively.

The acid addition salts of said basic compounds are prepared either by dissolving the free base in aqueous or aqueous alcohol solution or other suitable solvents containing the appropriate acid and isolating the salt by evaporating the solution, or by reacting the free base and acid in an organic solvent, in which case the salt separates directly or can be obtained by concentration of the solution.

The compounds of the invention may contain asymmetric carbon atoms apart from those in the sugar moiety. The invention includes the individual diastereomers and mixtures thereof. The individual diastereomers may be prepard or isolated by methods known in the art.

A preferred embodiment of the present invention is a compound of the Formula I, wherein R is of the Formula Ia and X, Y, Q, $R_2'$, $R_3'$, and $R_5'$ are as defined above or a pharmaceutically acceptable salt thereof.

Another preferred embodiment is a compound of Formula I wherein R is of the Formula Ia, X, and Y are hydrogen, and Q, $R_2'$, $R_3'$, and $R_5'$ are as defined above.

Another preferred embodiment is a compound of formula I wherein R is of the Formula Ia, X, and Y are hydrogen, and Q is 5′-deoxy-β-D-ribose, 5′-deoxy-5-′-methylthio ribose, or 5′-deoxy-5′-chloro ribose and $R^{2\prime}$ and $R_3'$ are each independently hydrogen, acetyl, benzoxyl, benzoyl substituted by lower alkyl, lower alkoxy, halogen, or when taken together are alkylidene, particularly popropylidene.

A particular embodiment includes 5′-deoxy-$N^6$-(2,2-diphenylethyl)adenosine or a phamaceutically acceptable salt thereof.

A particular embodiment includes 5′-deoxy-5′-methylthio-$N^6$-(2,2-diphenylethyl)adenosine or a pharmaceutically acceptable salt thereof.

A particular embodiment includes 5′-deoxy-5′-chloro-$N^6$-(2,2-diphenylethyl)adenosine or a pharmaceutically acceptable salt thereof.

A second generic embodiment of the present invention is a compound of the Formula I, wherein R is of the Formula Ib and X, Y, Q, $R_2$, $R_3$, and $R_5$ are as defined above or a pharmaceutically acceptable salt thereof.

Another second generic preferred embodiment is a compound of Formula I where R is of the Formula Ib, X and Y are hydrogen, and Q, $R_2'$, $R_3'$, and $R_5'$ are as defined above.

Still another second generic preferred embodiment is a compound of Formula I wherein R is of the Formula Ib, X and Y are hydrogen, and Q is 5′-deoxy-β-D-ribose, 5′-deoxy-5′-methylthio ribose, or 5′-deoxy-5′-chloro ribose or acetyl esters thereof and $R_2'$ and $R_3'$ are each independently hydrogen, acetyl, benzoyl, or when taken together are isopropylidene.

A particular embodiment of the compounds of Formula I wherein $R_1$ is of the Formula Ib includes 5′-deoxy-$N^6$-(1-indanyl)adenosine or a pharmaceutically acceptable salt thereof.

A particular embodiment includes 5′-deoxy-5′-methylthio-$N^6$-(1-indanyl)adenosine or a pharmaceutically acceptable salt thereof.

A particular embodiment includes 5′-deoxy-5′-chloro-$N^6$-(1-indanyl)-adenosine or a pharmaceutically acceptable salt thereof.

A third generic embodiment of the present invention is a compound of the Formula I, wherein R is of the Formula Ic and X, Y, Q, $R_2'$, $R_3''$, and $R_5'$ are as defined above or a pharmaceutically acceptable salt thereof.

Another preferred third generic embodiment is a compound of Formula I wherein R is of the Formula Ic, X and Y are hydrogen, and Q, $R_2'$, $R_3'$, and $R_5'$ are as defined above.

Still another preferred third generic embodiment is a compound of Formula I where R is of the Formula Ic, X and Y are hydrogen, and Q is 5′-deoxy-β-D-ribose, 5′-deoxy-5′-methylthio ribose, or 5′-deoxy-5′-chlororibose or acetyl esters thereof and $R_2'$ and $R_3'$ are each independently hydrogen, acetyl, benzoyl or when taken together are isopropylidene.

A particular embodiment of the compounds of the Formula I wherein R is of the Formula Ic includes 5′-deoxy-$N^6$-(1-methyl-2-phenylethyl)adenosine or a pharmaceutically acceptable salt thereof.

A particular embodiment includes 5′-deoxy-5′-methylthio-$N^6$-(1-methyl-2-phenylethyl)adenosine or a pharmaceutically acceptable salt thereof.

A particular embodiment includes 5′-deoxy-5′-chloro-$N^6$-(1-methyl-2-phenylethyl)adenosine or a pharmaceutically acceptable salt thereof.

A fourth generic aspect of the present invention is a compound of the Formula I, wherein R is cycloalkyl having three to eleven ring members, and Q, $R_2'$, $R_3'$, and $R_5'$ are as defined above or a pharmaceutically acceptable salt thereof.

A preferred fourth generic aspect is a compound of Formula I wherein R is a 5—7 membered cycloalkyl radical, and Q, $R_2'$, $R_3'$, and $R_5'$ are as defined above.

Another preferred fourth generic aspect is a compound of Formula I wherein R is a 5—7 membered cycloalkyl radical, and Q is 5′-deoxy-β-D-ribose, 5′-deoxy-5′-methylthio ribose, or 5′-deoxy-5′-chloro ribose or acetyl esters thereof and $R_2'$ and $R_3'$ are each independently hydrogen, acetyl, benzoyl, or when taken together are isopropylidene.

A particular embodiment of the compounds of the Formula I wherein R is of the Formula Id is 5′-deoxy-$N^6$-(cyclopentyl)adenosine or a pharmaceutically acceptable salt thereof.

A particular embodiment includes 5′-deoxy-5′-methylthio-$N^6$-(cyclopentyl)adenosine or a pharmaceutically acceptable salt thereof.

$R_3'$, and $R_5'$ are as defined above or a pharmaceutically acceptable salt thereof.

A preferred fourth generic aspect is a compound of Formula I wherein R is a 5—7 membered cycloalkyl radical, and Q, $R_2'$, $R_3'$, and $R_5'$ are as defined above.

Another preferred fourth generic aspect is a compound of Formula I wherin R is a 5—7 membered cycloalkyl radical, and Q is 5′-deoxy-β-D-ribose, 5′-deoxy-5′-methylthio ribose, or 5′-deoxy-5′-chloro ribose or acetyl esters thereof and $R_2'$ and $R_3'$ are each independently hydrogen, acetyl, benzoyl, or when taken together are isopropylidene.

A particular embodiment of the compounds of the Formula I wherein R is of the Formula Id is 5′-deoxy-$N^6$-(cyclopentyl)adenosine or a pharmaceutically acceptable salt thereof.

A particular embodiment includes 5′-deoxy-5′-methylthio-$N^6$-(cyclopentyl)adenosine or a pharmaceutically acceptable salt thereof.

A particular embodiment includes 5′-deoxy-5′-chloro-$N^6$-(cyclopentyl)adenosine or a pharmaceutically acceptable salt thereof.

A fifth generic embodiment of the present invention is a compound of the Formula I, wherein R is of the Formula Ie and X, Y, Q, $R_2$, $R_3$, and $R_5$ are as defined above or a pharmaceutically acceptable salt thereof.

Another fifth generic preferred embodiment is a compound of Formula I wherein R is of the Formula Ie,

X and Y are hydrogen, and Q, $R_2'$, $R_3'$, and $R_5'$ are as defined above.

Still another fifth generic preferred embodiment is a compound of Formula I where R is of the Formula Ie, X and Y are hydrogen, and Q is 5'-deoxy-β-D-ribose, 5'-deoxy-5'-methylthio-ribose, or 5'-deoxy-5'-chloro ribose or acetyl esters thereof and $R_2'$ and $R_3'$ are each independently hydrogen, acetyl, benzyl, or when taken together are isopropylidene.

A particular embodiment of the compounds of Formula I wherein $R_1$ is of the Formula Ie includes $N^6$-α-naphthylmethyl-5'-deoxyadenosine or $N^6$-α-naphthylmethyl-5'-deoxyadenosine-2',3'-di-O-acetyl.

The compounds of Formula I may be conveniently synthesized by reacting the appropriate N-6-substituted purine II with the protected deoxy sugar III, IIIa, or IIIb as follows:

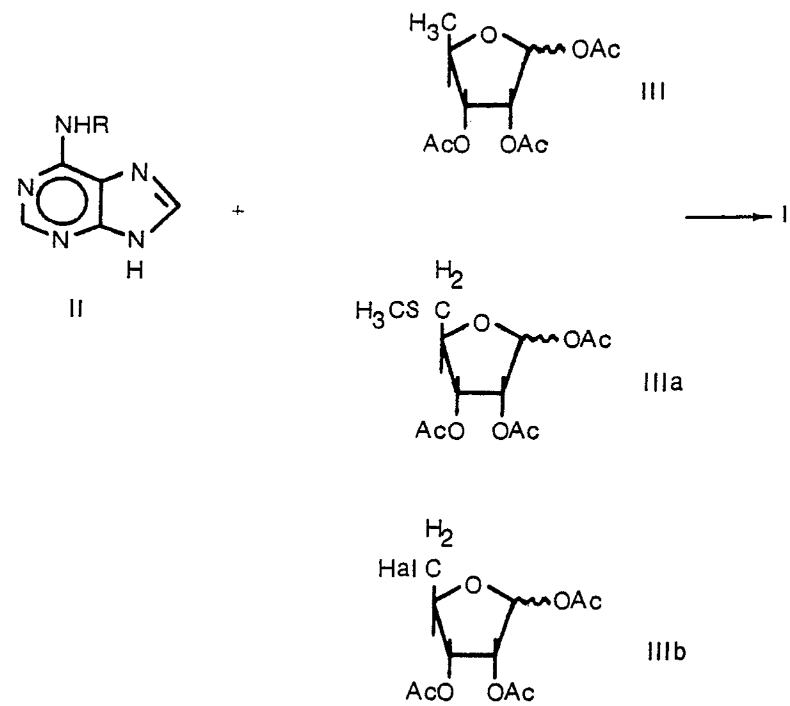

II + III, IIIa, IIIb → I

Compounds of Formula II where R is as defined above is reacted with compound of Formula III, IIIa, or IIIb in a neat melt and heating for approximately 1 to 12 hours. Addition of an acid in a catalytic amount is useful.

The compounds of Formula IIIb may be prepared by the following scheme:

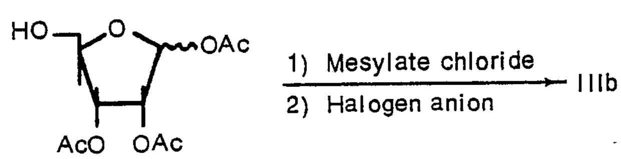


They may also be synthesized from the known 6-chloropurineriboside as shown in Scheme I.

SCHEME I

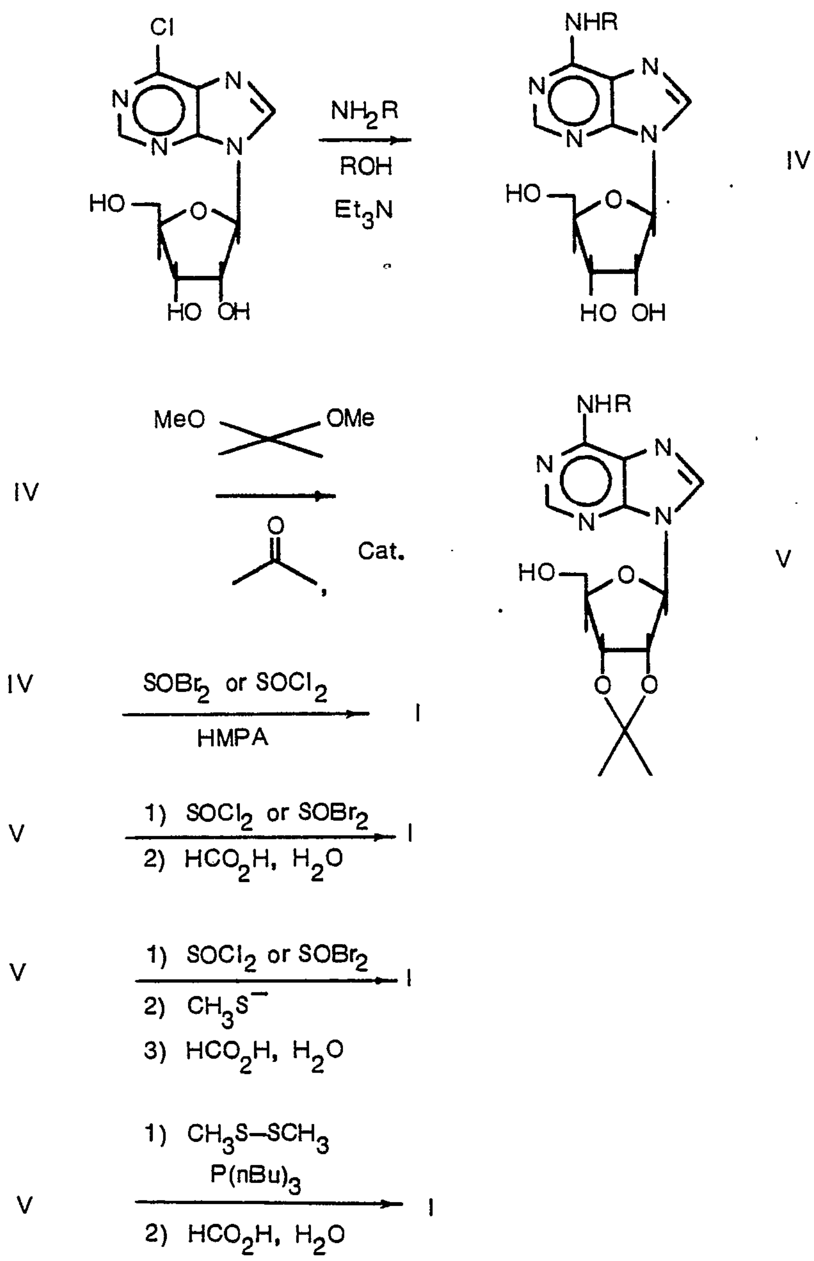


Compounds of Formula IV are made by reacting 6-chloropurine-riboside with the amine $NH_2R$ where R is as defined above, in an inert solvent and in the presence of a base such as triethylamine. Compound IV can be converted to the isopropylidene analog V by reacting with 2,2-dimethoxypropane in acetone and in the presence of an acid catalyst such as bis-(p-nitrophenyl)phosphate hydrate (Hampton's reagent). The 5'-deoxy-5'-chloro and 5'-deoxy-5'-bromo compounds of Formula I are synthesized by reacting the appropriate Compound V with thionyl chloride or thionyl bromide followed by treatment with formic acid and water to remove the isopropylidene and give Compound I. Compounds of Formula I may also be obtained by directly treating IV with thionyl chloride or thionyl bromide in hexamethyl phosphoamide (HMPA).

The 5'-deoxy-5'-methylthio adenosine compounds are prepared in one of two ways. The $N^6$-substituted 5'-deoxy-5'-chloro adenosine isopropylidene compound can be treated with thiomethoxide to displace the chloro moiety, followed by formic acid-water deprotection to yield compounds of Formula I. alternatively, a compound of Formula V can be treated with dimethyldisulfide in the presence of tri-n-butylphosphine, followed by formic acid-water deprotection, to give compounds of Formula I where Z is $CH_2SCH_3$.

Additionally, the compounds of Formula I wherein Z is F may be prepared by the following reaction:

(1) Mesyl Chloride
V ⟶ I
(2) $F^-$
(3) $HCO_2H/H_2O$

The compounds of Formula I have been found to possess differing affinities for adenosine receptors (designated $A_1$ and $A_2$ receptors for convenience). These compounds are active in animal tests which are predictive of neuroleptic activity for the treatment of major psychoses such as schizophrenia. These compounds also have analgesic properties and as such, are useful in the treatment of pain.

In addition, the compounds of the present invention are useful as antihypertensive agents for the treatment of high blood pressure.

## PHARMACOLOGICAL EVALUATION

### Adenosine Receptor Binding — $A_1$ Receptor Affinity (RBA1)

Preparation of Membranes

While brain minus cerebellum and brainstem from male Long Evans rats (150—200 g) was homogenized in 30 volumes of ice-cold 0.05 M Tris-CHI buffer pH 7.7 using a Brinkman Polytron PT—10, (setting number 6 for 20 seconds) and centrifuged for ten minutes at 20,000 × g (Sorvall RC—2), 4°C. The supernatant was discarded, and the pellet was resuspended and centrifuged as before. The pellet was resuspended in 20 ml Tris-HCl buffer containing two International Units/ml of adenosine deaminase (Sigma type III from calf intestinal mucosa), incubated at 37°C for 30 minutes, then subsequently at 0°C for ten minutes. The homogenate was again centrifuged, and the final pellet was resuspended in ice-cold 0.05 M Tris-HCl buffer pH 7.7 to a concentration of 20 mg/ml original wet tissue weight and used immediately.

Assay Conditions

Tissue homogenate (10 mg/ml) was incubated in 0.05 M Tris-HCl buffer pH 7.7 containing 1.0 nM ($^3H$)-$N^6$-cyclohexyladenosine ([$^3H$]—CHA) with or without tests agents in triplicate for one hour at 25°C. Incubation volume was 2 ml. Unbound [$^3H$]-CHA was separated by rapid filtration under reduced pressure through Whatman glass fiber (GF/B) filters. The filters were rinsed three times with 5 ml of ice cold 0.05 M Tris-HCl buffer pH 7.7. The radio-labeled ligand retained on the filter was measured by liquid scintillation spectrophotometry after shaking the filters for one hour or longer on a mechanical shaker in 10 ml of Beckman Ready-Solv HP scintillation cocktail.

Calculations

Nonspecific binding was defined as the binding which occurred in the presence of 1 mM theophylline. The concentration of test agent which inhibited 50% of the specific binding ($IC_{50}$) was determined by nonlinear computer curve fit. The Scatchard plot was calculated by linear regression of the line obtained by plotting the amount of radioligand bound (pmoles/gram of tissue)

$$\text{versus } \left[\frac{\text{bound radioligand}}{\text{free radioligand}}\right].$$

Since the amount of radioligand bound was a small fraction of the total amount added, free radioligand was defined as the concentration (nM) of radioligand added to the incubation mixture. The Hill coefficient was calculated by linear regression of the line obtained by plotting the log of the bound radioligand vs the log of the

$$\left[\frac{\text{bound radioligand}}{B_{max} - \text{bound radioligand}}\right].$$

The maximal number of binding sites ($B_{max}$) was calculated from the Scatchard plot.

### Adenosine Receptor Binding — $A_2$ Receptor Affinity (RBA2)

Tissue Preparation

Brains from 200—500 g mixed sex Sprague-Dawley rats were purchased from Pel-Freez (Rogers, Arkansas). Fresh brains from male Long-Evans hooded rats (Blue Spruce Farms, Altamont, NY) gave essentially indentical results. Brains were thawed and then kept on ice while the striata were dissected out. Striata were disrupted in 10 vol of ice-cold 50 mM Tris·HCl (pH 7.7 at 25°C, pH 8.26 at 5°C) (Tris) for 30

seconds in a Polytron PT—10 (Brinkmann) at setting 5. The suspension was centrifuged at 50,000 xg for ten minutes, the supernatant discarded, the pellet resuspended in 10 vol ice-cold Tris as above, recentrifuged, resuspended at 1 g/5 ml, and stored in plastic vials at −70°C (stable for at least six months). When needed, tissue was thawed at room temperature, disrupted in a Polytron, and kept on ice until used.

Incubation Conditions

All incubations were for 60 minutes at 25°C in 12×75 mm glass tubes containing 1 ml Tris with 5 mg original tissue weight of rat weight of rat striatal membranes, 4 nM [$^3$H]-N-ethyl adenosine-5′-carboxamide ([$^3$H]NECA), 50 nM $N^6$-cyclopentyladenosine (to eliminate $A_1$ receptor binding), 10 mM $MgCl_2$, 0.1 units/ml, of adenosine deaminase and 1% dimethylsulfoxide. $N^6$-Cyclopentyladenosine was dissolved at 10 mM in 0.02 N HCl and diluted in Tris. Stock solutions and dilutions of $N^6$-cyclopentyladenosine could be stored at −20°C for several months. Test compounds were dissolved at 10 mM in dimethylsulfoxide or the same day as the experiment, and diluted in dimethylsulfoxide to 100x the final incubation concentration. Control incubations received an equal volume (10 µl) of dimethylsulfoxide; the resulting concentration of dimethylsulfoxide had no effect on binding. [$^3$H]NECA was diluted to 40 nM in Tris. The membrane suspension (5 mg/0.79 ml) contained sufficient $MgCl_2$ and adenosine deaminase to give 10 mM and 0.1 units/ml, respectively, final concentration in the incubation. For test compounds with $IC_{50}$ values less than 1 µM, the order of additions was test compound (10 µl), $N^6$-cyclopentyladenosine (100 µl), [$^3$H]NECA (100 µl), and membranes (0.79 ml). For test compounds with $IC_{50}$ values greater than 1 µM and limited water solubility, the order of additions (same volumes) was test compound, membranes, $N^6$-cyclopentyladenosine, and [$^3$H]NECA. After all additions, the rack of tubes was vortexed, and the tubes were then incubated for 60 min at 25°C in a shaking water bath. The rack of tubes was vortexed an additonal time halfway through the incubation.

Incubations were terminated by filtration through 2.4 cm GF/B filters under reduced pressure. Each tube was filtered as follows: the contents of the tube were poured onto the filter, 4 ml of ice-cold Tris were added to the tube and the contents poured onto the filter, and the filter was washed twice with 4 ml of ice-cold Tris. The filtration was complete in about twelve seconds. Filters were put in scintillation vials, 8 ml of Formula 947 scintillation fluid added, and the vials left overnight, shaken, and counted in a liquid scintillation counter at 40% efficiency.

Data Analysis

Nonspecific binding was defined as binding in the presence of 100 µM $N^6$-cyclopentyladenosine, and specific binding was defined as total binding minus nonspecific binding. The $IC_{50}$ was calculated by weighted nonlinear least squares curve-fitting to the mass-action equation.

$$Y = T - S \cdot \frac{D}{D + K}$$

where Y is cpm bound
T is cpm total binding without drug
S is cpm specific binding without drug
D is the concentration of drug
and K is the $IC_{50}$ of the drug

Weighting factors were calculated under the assumption that the standard deviation was proportional to the predicted value of Y. Nonspecific binding was treated as a very large (infinite) concentration of drug in the computer analysis.

The $IC_{50}$ values (nM) for adenosine $A_1$ and $A_2$-receptor affinity are reported in the table.

| Example Number | Receptor Binding RBA—1 (nM) | RBA—2 (nM) |
|---|---|---|
| 1 | 227 | 1110 |
| 2 | 1680 | 21200 |
| 3 | 40 | 5700 |
| 4 | 556 | 20000 |
| 5 | 16 | 17200 |
| 6 | 52 | 21400 |
| 7 | 51 | 6300 |
| 8 | 700 | 44000 |
| 9 | 9 | 7400 |
| 10 | 13 | 7200 |
| 11 | 660 | 2000 |
| 12 | 1490 | 58200 |
| 13 | 53 | 240 |
| 14 | 218 | 8650 |
| 15 | 3 | 1600 |
| 16 | 35 | 7900 |
| 17 | 2 | 2700 |
| 18 | 1.2 | 2800 |
| 19 | 1370 | 71300 |
| 20 | 1010 | 323 |
| 21 | 2520 | NT* |
| 22 | 607 | NT |
| 23 | 80 | 3040 |

*NT = Not tested

ANTIPSYCHOTIC EVALUATION

The compounds of the invention are new chemical substances which are useful as pharmaceutical agents for the treatment of psychoses. The antipsychotic activity of representative compounds of the invention was established by the Mouse Activity and Screen Test Procedure (MAST) described below.

Animals

Nine unfasted Swiss-Webster male mice weighing 20—30 g are equally divided into three groups for each drug dose to be tested. That is, data for each dose level was generated by three separate groups of three mice each.

Drugs

A minimum of three dose levels (10, 30, and 100 mg/kg) are tested for each drug. Treatments are administered intraperitoneally one hour prior to testing. All dosages are calculated as parent compound and given in volumes of 10 ml/kg. Compounds are dissolved or suspended in 0.2% Methocel. Control animals are injected with Methocel.

Testing

A two part testing procedure is started one hour post injection. First, the screen test (ST) is performed (see *Pharmac. Biochem. Behav. 6*, 351—353, 1977). Briefly this test consists of placing mice on individual wire screens which are then rotated 180 degrees at the start of a 60 second observation period. The number of mice falling off the inverted screen is recorded.

Immediately following the screen test, the final phase of testing is initiated by placing each group of three mice in one actophotometer (*Life Sciences*, *22*, 1067—1076, 1978). The actophotometer consists of a cylindrical chamber whose center is occupied by another cylinder which contains the illumination for six photocells located on the perimeter of the chamber. Six light-beam interruptions equal one count. Locomotor activity is recorded by computer at ten minutes intervals for 60 minutes.

Data

The data obtained from the screen test are expressed as percent of mice falling off the screen. Data derived from locomotor activity of drug treated mice are compared to the activity of vehicle treated animals and are expressed as percent inhibition of spontaneous locomotion. All percentages reported for inhibition of locomotion (LI) are based upon data accumulated for one hour. Both phases of testing are graded: A=60—100%; C=31—59%; and N=0—30%. An overall dose rating is obtained by the following criteria:

| Inhibition of Locomotion Rating | with | Screen Test Failure Rating | = | Dose Rating |
|---|---|---|---|---|
| A | — | N or C | = | A |
| A | — | A | = | C |
| C | — | N or C | = | C |
| | | All other combinations | = | N |

LAD refers to the lowest dose at which an A rating is achieved. Compounds which exhibit an overall dose rating of A at a dose of 100 milligrams/kilogram or less are considered active. Utilizing this procedure, an overall dose rating of A was obtained for the noted compound at the indicated dose. The compounds are identified in the Examples.

| Example | Dose (mg/kg) | Inhibition of Mouse Locomotor Activity | Screen Test Failure |
|---|---|---|---|
| 1 | 3 | 29% | 11% |
| | 10 | 30% | 0% |
| | 30 | 65% | 11% |
| 2 | 10 | 16% | 0% |
| | 30 | 52% | 0% |
| | 100 | 80% | 11% |
| 3 | 10 | 19% | 0% |
| | 30 | 68% | 22% |
| | 100 | 96% | 100% |
| 4 | 10 | −16% | 0% |
| | 30 | 20% | 0% |
| | 100 | 56% | 0% |
| 5 | 1 | 25% | 0% |
| | 3 | 58% | 11% |
| | 10 | 69% | 22% |
| | 30 | 96% | 100% |
| | 100 | 99% | 100% |
| 6 | 1 | 12% | 0% |
| | 3 | 49% | 0% |
| | 10 | 83% | 88% |
| | 30 | 92% | 99% |

| Example | Dose (mg/kg) | Inhibition of Mouse Locomotor Activity | Screen Test Failure |
|---|---|---|---|
| 7 | 10 | 9% | 0% |
| | 30 | −50% | 0% |
| | 100 | 38% | 0% |
| 8 | 10 | 22% | 11% |
| | 30 | 39% | 0% |
| | 100 | 29% | 0% |
| 9 | 0.3 | 1% | 0% |
| | 1.0 | 46% | 0% |
| | 3.0 | 36% | 22% |
| | 10 | 90% | 77% |
| | 30 | 92% | 99% |
| | 100 | 97% | 99% |
| 10 | 10 | 41% | 0% |
| | 30 | 78% | 55% |
| | 100 | 99% | 99% |
| 11 | 10 | −4% | 0% |
| | 30 | 34% | 0% |
| | 100 | 90% | 0% |
| 12 | 3 | 44% | 0% |
| | 10 | 37% | 0% |
| | 30 | 59% | 0% |
| 13 | 10 | −13% | 0% |
| | 30 | 39% | 11% |
| | 100 | 63% | 0% |
| 14 | 10 | 57% | 0% |
| | 30 | 69% | 0% |
| | 100 | 96% | 0% |
| 15 | 10 | 65% | 67% |
| | 30 | 96% | 100% |
| | 100 | 100% | 100% |
| 16 | 10 | 2% | 0% |
| | 30 | 51% | 11% |
| | 100 | — | — |
| 17 | 0.3 | 44% | 11% |
| | 1.0 | 83% | 55% |
| | 3.0 | 95% | 99% |
| | 10 | 89% | 99% |
| | 30 | 100% | 99% |
| | 100 | 100% | 99% |
| 18 | 1.0 | 12% | 0% |
| | 0.3 | 25% | 11% |
| | 1.0 | 39% | 11% |
| | 3.0 | 52% | 22% |
| | 10 | 93% | 78% |
| | 30 | 94% | 100% |
| | 100 | 98% | 100% |

| Example | Dose (mg/kg) | Inhibition of Mouse Locomotor Activity | Screen Test Failure |
|---|---|---|---|
| 19 | 3 | 36% | 11% |
| | 10 | 55% | 0% |
| | 30 | 83% | 0% |
| 20 | 3 | −1% | 0% |
| | 10 | 49% | 0% |
| | 30 | 81% | 0% |
| 23 | 0.3 | −2% | 0% |
| | 1 | 0% | 0% |
| | 1 | 7% | 0% |
| | 3 | 48 | 0% |
| | 10 | 76 | 0% |
| | 30 | 81 | 0% |

A representative compound of the invention (identified in the Example 5) was also tested for antipsychotic activity according to the following protocol (SIDR). The noted compound has the indicated $ED_{50}$ values (mg/kg) and is considered active as an antipsychotic agent in the test procedure.

Procedure

Mature male Long-Evans rats or squirrel-monkeys are conditioned to push a lever in order to avoid a painful electric footshock. If the animal fails to push the lever, he receives a shock every ten seconds until the lever is pushed. Shocks can be terminated by pushing the lever. Thereafter, as long as the lever is pushed at least once every 20 seconds, there will be no shock.

Each animal acts as its own control; one weekly session is used to establish baseline behavior and another session later in the week is used as a drug session. Once patterns of avoidance are established, the effects of standard and unknown compounds are studied.

The compound (Example 5) had an $ED_{50}$ of 2.8 mg/kg for blockade of avoidance with a response of 10% at 4 mg/kg for blockade of escape.

ANTIHYPERTENSIVE EVALUATION (AHP3)

The usefulness of the compounds of the present invention as antihypertensive agents is demonstrated by their effectiveness in standard pharmacological test procedures, for example, in causing a significant decrease in mean arterial blood pressure in the conscious rat. This test procedure is described in the following paragraphs.

A Method for the Direct Monitoring of Aortic Blood
Presure and Heart Rate from Conscious Rats

The continuous monitoring of pulsatile blood pressure (BP) from unrestrained conscious rats surgically equipped with polyethylene cannulas was accomplished by means of a computer assisted data capture scheme (CADCS). The basic elements of the methodology are the cannulation procedure and the CADCS.

Method

*Cannulation Procedure:* Rats were anesthetized with Telazol (1:1 tiletamine HCl and zolazepam HCl); 20—40 mg/kg IM and the descending aorta exposed via a midline incision. Cannulas fabricated from polyethylene tubing were inserted into the aorta via an undersized puncture hole below the renal arteries. The puncture hole was made by a 23 G disposable needle with a section of the aorta clamped off above and below the puncture site. The cannulas, consisting of a PE100 (0.86 mm ID) body and a PE50 (0.58 mm ID) tip, were attached to a trocar, inserted through the psoas muscle, and passed subcutaneously along the midline of the back and externalized between the ears. The cannulas were anchored to the psoas muscle and between the scalulae (3—0 green braided suture). The dline incision was closed in two steps (muscle first, skin second) using continuous over-and over sutures (4—0 chronic). Each rat was then given penicillin 30,000 units subcutaneously (Penicillin G Procaine Sterile Suspension).

The rats were fitted with a harness-spring-swivel assembly designed to protect the cannula and to provide the rat relative freedom of movement. The harnesses were fabricated from nylon hook and loop

tape cemented to a metal plate to which spring wires (18—8 stainless steel), were attached to brass swivels. Each polyethylene cannula was channeled through a spring and connected through a swivel to a pressure transducer (Model P23Gb; Statham Instruments; Hato Rey, Puerto Rico) and an infusion pump (Sage model 234—7; Orion Research, Cambridge, MA) by means of PE100 tubing. While on test, each rat received a continuous slow infusion of heparinized saline solution (approximately 400 l or 40 units of heparin per 24 hour period) to prevent clot formation. Additional "flushes" of the cannula with heparinized saline were carried out when the aortic pulse pressure (systolic minus diastolic) was less than 25 mm Hg.

*CADCS*: The pulsatile blood pressure and heart rate of each of 32 rats was monitored every minute by means of two in-laboratory microcomputers communicating directly with a data concentrator computer. The data were first stored on the data concentrator disk and then tranferred to a magnetic tape for analysis and report generation by the main research computer. The overall scheme involved modulating the primary signal from the pressure transducer, generating the primary data set of the one-minute values for systolic, diastolic, and mean blood pressures and heart rate by the in-lab microcomputer and the storage, analysis, and report generation by the main research computer.

The transducers were connected to analog signal conditioning modules. The modules provided a regulated excitation voltage for the transducers, amplification as required to interface the microprocessors and an active low pass filter to compensate for the pressure wave form distortion produced by the flexible, fluid filled, narrow cannula. The distortion was 22—26 Hz and this provided a reliable estimate of both systolic and diastolic blood pressure.

The microcomputers (one for each of two groups of 16 rats) were connected to the input components through the module interface units, an analog-to-digital converter for the pressure wave form signal and the digital inputs for the dose and event marker switches. The microcomputer controlled the sequential acquision of data from the modular interface units through an internal synchronous time-of-day clock/time base generator. Utilizing the time base generator as a reference, the blood pressure values and the marker switch status for each of the 32 stations were sampled every ten msec. The microcomputer processed each blood pressure sample as it was received to produce "running average" values for heart rate, and mean, systolic and diastolic blood pressures.

When tested by the above procedure, the following compounds produced the following changes in MAP (mean arterial pressure) and heart rate.

| Example Number | mg/kg | | Hour 1 | 3 | 5 | 7 | 9 |
|---|---|---|---|---|---|---|---|
| 2 | 10 | MAP | ↓18% | ↓13 | ↑ 4 | ↓ 5 | ↓ 9 |
| | | HR | ↓10% | 0 | ↑ 7 | ↑14 | ↑12 |
| 3 | 10 | MAP | ↓17 | ↓ 9 | ↓ 6 | ↓11 | ↓ 6 |
| | | HR | ↓18 | ↓ 9 | ↓ 6 | ↓ 5 | ↓10 |
| 4 | 10 | MAP | ↓ 2 | ↓ 3 | ↓15 | ↓ 1 | ↓ 8 |
| | | HR | ↑ 3 | ↑ 9 | ↓ 8 | ↑17 | ↑ 7 |
| 5 | 10 | MAP | ↓36 | ↓23 | ↓20 | ↓19 | ↓18 |
| | | HR | ↓65 | ↓60 | ↓46 | ↓39 | ↓41 |
| 7 | 10 | MAP | ↑8% | ↑5% | ↑8% | ↑10% | ↓5% |
| | | HR | ↑4% | ↑5% | ↑16% | ↑25% | ↑12% |
| | 30 | MAP | ↓24% | ↓14% | ↓4% | ↑1% | 0% |
| | | HR | ↓27% | ↓9% | ↑7% | ↑22% | ↑14% |
| 8 | 10 | MAP | ↑1% | ↑10% | ↑2% | ↑3% | ↑4% |
| | | HR | ↑9% | ↑14% | ↑6% | ↑17% | ↑17% |
| | 30 | MAP | ↓5% | ↓5% | ↓5% | ↓8% | ↓5% |
| | | HR | ↓5% | ↑1% | ↑2% | ↓6% | ↓3% |
| 9 | 10 | MAP | ↓20% | ↓25% | ↓13% | ↓13% | ↓7% |
| | | HR | ↓46% | ↓43% | ↓33% | ↓21% | ↓4% |

| Example Number | mg/kg | | Hour 1 | 3 | 5 | 7 | 9 |
|---|---|---|---|---|---|---|---|
| 10 | 3 | MAP | ↓11% | 0% | ↓7% | ↓7% | ↓7% |
| | | HR | ↓29% | ↓11% | ↓6% | ↑15% | ↑20% |
| | 10 | MAP | ↓21% | ↓13% | ↓5% | ↓9% | ↓6% |
| | | HR | ↓43% | ↓28% | ↓13% | ↓9% | ↓6% |
| 11 | 10 | MAP | ↑3% | ↑2% | ↑2% | 0% | ↑7% |
| | | HR | ↑1% | ↓6% | ↑6% | ↑1% | ↑10% |
| 12 | 10 | MAP | ↓16% | ↓4% | ↓7% | ↓6% | ↓1% |
| | | HR | ↓19% | ↑9% | 0% | ↓6% | ↑4% |
| 14 | 10 | MAP | ↓12% | ↓9% | ↑1% | ↓7% | ↓2% |
| | | HR | ↓23% | ↓9% | 0% | ↓3% | ↑17% |
| 15 | 10 | MAP | ↓24% | ↓33% | ↓29% | ↓26% | ↓14% |
| | | HR | ↓44% | ↓55% | ↓53% | ↓42% | ↓29% |
| 16 | 10 | MAP | ↓14% | ↓13% | ↓14% | ↓13% | ↓6% |
| | | HR | ↓23% | ↓20% | ↓12% | ↓10% | ↑2% |
| 17 | 10 | MAP | ↓42% | ↓41% | ↓37% | ↓36% | ↓32% |
| | | HR | ↓64% | ↓64% | ↓61% | ↓58% | ↓53% |
| 18 | 10 | MAP | ↓43% | ↓30% | ↓23% | ↓26% | ↓23% |
| | | HR | ↓63% | ↓58% | ↓47% | ↓45% | ↓40% |
| 19 | 10 | MAP | ↓32% | ↓20% | ↓20% | ↓20% | ↓12% |
| | | HR | ↓6% | ↓4% | ↑4% | ↑5% | ↑1% |
| 20 | 10 | MAP | ↓16% | ↓15% | ↓15% | ↓15% | ↓15% |
| | | HR | ↑15% | ↑19% | ↑13% | ↑15% | ↑9% |
| 21 | 10 | MAP | 0% | ↓11% | ↓15% | ↓18% | ↓9% |
| | | HR | ↑23% | ↑13% | ↑3% | ↑8% | ↑14% |
| 22 | 10 | MAP | ↓21% | ↓8% | ↓17% | ↓17% | ↓20% |
| | | HR | ↑7% | ↑23% | ↑8% | ↑14% | ↑8% |
| 23 | 10 | MAP | ↓3% | ↓5% | ↓4% | ↓4% | ↓16% |
| | | HR | ↓19% | ↓11% | ↓6% | ↓8% | ↓15% |

## ANALGESIC EVALUATION

The antiwrithing (AW) test provides preliminary assessment of compounds with potential analgesic activity. The test is performed in male Swiss-Webster mice. Compounds are administered subcutaneously in aqueous 0.2% methylcellulose or other appropriate vehicles in volumes of 10 ml/kg. Dosages represent active moiety.

Acetic acid: 0.6%, 10 ml/kg) is injected intraperitoneally 20 minutes after administration of the adenosine agonist. Writhing movements are counted for five minutes starting seven minutes after the acetic acid injection. Writhing is defined as abdominal constriction and stretching of the body and hind legs with concave arching of the back. Data are expressed as $ED_{50}$ values, where the $ED_{50}$ is the dose necessary to suppress writhing by 50% relative to vehicle controls. $ED_{50}$ values are calculated by nonlinear regression analysis. For example, 5'-deoxy-$N^6$-cyclopentyladenosine, a representative compound shown as Example 5 of the present invention, had an $ED_{50}$ of 0.9 mg/kg when administered intraperitoneally to mice.

Also when administered intraperitoneally, the compound of Example 12 had an $ED_{50}$ of 3.0 mg/kg and 5'-chloro-$N^6$-(R)phenylisopropyl adenosine (Example 15) had an $ED_{50}$ of 1.8 mg/kg when administered intraperitoneally.

Accordingly, the present invention also includes a pharmaceutical composition for treating psychoses, pain or hypertension comprising a corresponding antipsychotic, analgesic or antihypertensive effective amount of a compound of the Formula I as defined above together with a pharmaceutically acceptable carrier.

For preparing pharmaceutical compositions from the compounds described by this invention, inert, pharmaceutically acceptable carriers can be either solid or liquid. Solid form preparations include powders, tablets, dispersible granules, capsules, cachets, and suppositories. A solid carrier can be one or more substances which may also act as diluents, flavoring agents, solubilizers, lubricants, suspending agents, binders or tablet disintegrating agents; it can also be encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 to 10 to about 70 percent of the active ingredient. Suitable solid carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets, and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogenoeus mixture is then poured into convenient sized molds, allowed to cool and thereby to solidify.

Liquid form preparations include solutions, suspensions, and emulsions. As an example may be mentioned water or water propylene glycol solutions for parenteral injection. Liquid preparations can also be formulated in solution in aqueous polyethylene glycol solution. Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, i.e., natural or synthetic gums, resins, methylcellulose, sodium carboxymethylcellulose, and other well-known suspending agents.

Also included are solid form preparations which are intended to be converted, shortly before use, to liquid form preparations for either oral or parenteral administration. Such liquid forms include solutions, suspensions, and emulsions. These particular solid form preparations are most conveniently provided in unit dose form and as such are used to provide a single liquid dosage unit. Alternately, sufficient solid may be provided so that after conversion to liquid form, multiple individual liquid doses may be obtained by measuring predetermined volumes of the liquid form preparation as with a syringe, teaspoon, or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavorants, colorants, stabilizers, buffers, artificial and natural sweeteners, dispersants, thickeners, solubilizing agents, and the like. The liquid utilized for preparing the liquid form preparation may be water, isotonic water, ethanol, glycerine, propylene glycol, and the like as well as mixtures thereof. Naturally, the liquid utilized will be chosen with regard to the route of administration, for example, liquid preparations containing large amounts of ethanol are not suitable for parenteral use.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules, and powders in vials or ampoules. The unit dosage form can also be a capsule, cachet, or tablet itself or it can be the appropriate number of any of these in packaged form.

The quantity of active compound in a unit dose of preparation may be varied or adjusted from 1 mg to 500 mg preferably to 5 to 100 mg according to the particular application and the potency of the active ingredient. The compositions can, if desired, also contain other compatible therapeutic agents.

In therapeutic use as described above, the mammalian dosage range for a 70 kg subject is from 0.1 to 150 mg/kg of body weight per day or preferably 1 to 50 mg/kg of body weight per day. The dosages, however, may be varied depending upon the requirements of the patent, the severity of the condition being treated, and the compound being employed. Determination of the proper dosage for a particular situation is within the skill of the art. Generally, treatment is initiated with smaller dosages which are less than the optimum dose of the compound. Thereafter the dosage is increased by small increments until the optimum effect under the circumstances is reached. For convenience, the total daily dosage may be dividced and administered in portions during the day if desired.

The following Examples further illustrate the invention.

## Example 1

$N^6$-[2,2-Diphenylethyl]-5'-deoxyadenosine

A mixture of the $N^6$-(2,2-diphenylethyl)purine (31.5 g, 10 mmol) and the triacetate sugar III* (2.6 g, 10 mmol) was heated at 180°C. To this was added one microdrop of $H_2SO_4$ and melt stirred at 180—195°C for four hours. The melt cooled to room temperature, solidified. The solid dissolved in ethyl acetate and purified by silica gel flash chromatography. The new compound was isolated by evaporation of solvent and

* Kiss, J., D'Souza, R., Koereringe, J. A., Arnold, W.; *Helv. Chim. Acta., 65,* (5), 1522—1537 (1982).

placed in a solution of methanol (70 mL) and sodium methoxide (0.05 g, 1 mmol). The mixture was stirred at room temperature for four hours. The reaction quenched with Dowex 50 (H+ form) to pH=6. The resin was removed by filtration and the solvent removed in vacu to give a hygroscopic foam. The foam was coevaporated to dryness with Methanol, yield 0.26 g (8%): mp 185—187.5.

Anal ($C_{24}H_{25}N_5O_3$);
calc: C=58.81, H=5.61, N=20.52,
found: C=59.00, H=5.32, N=19.62.

$^1$H NMR (DMSO-$d_6$, 200 MHz): δ 1.2 (d, 3H), δ 3.9 (m, 2H), δ 4.1 (bs, 2H), δ 4.6 (m, 2H), δ 5.2 (d, 1H), δ 5.4 (d, 1H), δ 5.8 (d, 1H), δ 7.1—7.3 (m, 10H), δ 7.8 (bs, 1H), δ 8.3 (bs, 2H).

The starting purine was prepared as follows: a solution of 2,2-diphenylethylamine (7 g, 35.5 mmol), triethylamine (3.6 g, 35.5 mmol) and 6-chloropurine (5.5 g, 35.5 mmol) in ethanol (250 mL) was stirred at reflux, overnight. The solution was cooled to room temperature. The precipitated solid was collected and washed with ethanol (100 mL). The off-white solid was dried in vacuo at room temperature; yield 6.5 g (58%): mp=232—233°C.

Anal ($C_{19}H_{17}N_5$),
calc: C=72.36, H=5.43, N=22.21,
found: C=72.05, H=5.42, N=21.99.

Example 2

N$^6$-[1-Indanyl]-5'-deoxyadenosine

The title compound was prepared as Example 1 using 2.5 g, 10 mmol of N$^6$-(1-indanyl)purine and 4.0 g, 15 mmol of the triacetate-5-deoxy sugar. The hydrolysis used 0.25 g of sodium methoxide and yielded 0.7 g (19%) as an extremely hygroscopic syrup.

Anal ($C_{19}H_{21}N_50_3$);
calc: C=62.11, H=5.76, N=19.06,
found: C=60.67, H=6.14, N=18.26.

$^1$H NMR (DMSO-$d_6$, 90 MHz): δ 1.25 (d, 3H), δ 2.0—2.2 (m, 2H), δ 2.8—3.1 (m, 2H), δ 3.8—4.0 (m, 2H), δ 4.6 (m, 1H), δ 5.05 (d, 1H), δ 5.3 (d, 1H), δ 5.8 (d, 1H), δ 5.8—6.0 (bs, 1H), δ 7.0—7.3 (m, 4H), δ 7.9 (br.d, 1H), δ 8.15 (s, 1H), δ 8.2 (s, 1H).

The starting purine was prepared as in Example 5 using 1-aminoindane (1.3 g, 10 mmol), triethylamine (1.1 g, 10 mmol) and 6-chloropurine (1.5 g, 10 mmol); yield 1.6 g (64%): mp=247—248°C*d.*

Anal ($C_{14}H_{13}N_5$),
calc: C=66.91, H=5.21, N=27.87,
found: C=66.46, H=5.26, N=27.54.

Example 3

(R)-N$^6$-[Phenylisopropyl]-5'-deoxyadenosine

The reaction proceeded as in Example 1 with N$^6$-[phenylisopropyl]purine (4.5 g, 17.8 mmol) and 4.6 g (17.8 mmol) of the deoxy-sugar. The hydrolysis used 0.3 g of sodium methoxide and yielded 1.8 g (28%) hygroscopic solid, mp=~70°C.

$^1$H NMR (DMSO-$d_6$), 200 MHz): δ 1.1 (d, 3H), δ 1.2 (d, 3H), δ 2.7—2.8 (m, 1H), δ 3.0 (m, 1H), δ 3.95 (m, 2H), δ 4.5 (m, 2H), δ 5.2—5.6 (br.s, 2H), δ 5.9 (d, 1H), δ 7.0—7.2 (m, 5H), δ 7.6 (br.d, 1H), δ 8.3 (s, 1H), δ 8.5 (s, 1H).

The starting purine was prepared as follows: a solution of the l-amphetamine (13.6 g, 0.1 mmol) and 6-chloropurine (7.73 g, 0.05 mmol) in ethanol (250 mL) was stirred at reflux for 72 hours. The solution cooled to 45°C and the ethanol was removed in vacuo. The residue was poured into a mixture of chloroform and water (100 mL). The organics were separated and dried over $MgSO_4$ and the solvent removed in vacuo to give a white solid which was dried in vacuo, at RT, overnight; yield 11 g (87%): mp=190—193°C.

Anal ($C^{14}H_{15}N_5$),
calc: C=66.38, H=5.97, N=27.65,
found: C=66.10, H=5.96, N=27.14.
$[\alpha]_D$=−114.8 (c 1.08, MeOH).

Example 4

(S)-N$^6$-[Phenylisopropyl]-5'-deoxyadenosine

The title compound was prepared as in Example 1 using 5.0 g, 19.7 mol of N$^6$-(S)-phenylisopropylpurine and 5.2 g, 19.7 mol of the triacetate-5-deoxy sugar. The hydrolysis used 0.3 g sodium methoxide and yielded 1.3 g (19%) as an extremely hygroscopic syrup.

Anal ($C_{19}H_{23}N_5O_3$);
calc: C=61.77, H=6.27, N=18.96,
found: C=60.52, H=6.52, N=17.28.

$^1$H NMR (DMSO-$d_6$, 200 MHz): δ 1.1 (d, 3H), δ 1.2 (d, 3H), δ (m, 1H), δ 2.8 (m, 1H), δ 2.9—3.0 (m, 1H), δ 3.95 (d, 2H), δ 4.6 (m, 2H), δ 5.8 (d, 1H), δ 7.0—7.2 (m, 5H), δ 8.1—8.2 (br.s, 2H), δ 8.3 (s, 1H).

The starting purine was prepared as described for the starting material in Example 3 using d-amphetamine (13.3 g, 0.098 mol) and 6-chloropurine (7.73 g, 0.05 mol); yield 8.7 g (69%): mp=190—192.5.

Anal ($C_{14}H_{15}N_5$),
calc: C=66.38, H=5.97, N=27.65,
found: C=66.20, H=6.01, N=27.52.
$[\alpha]_D$=+87.0 (c 1.19, MeOH).

Example 5

$N^6$-[Cyclopentyl]-5'-deoxyadenosine

The reaction proceeded as in Example 1 with 5.0 g, 24.6 mmol of $N^6$-cyclopentylpurine and 6.4 g, 24.6 mmol of the triacetate-5-deoxy sugar. The hydrolysis used 0.5 g of sodium methoxide and yielded, 2.6 g (33%) of extremely hygroscopic syrup.

Anal ($C_{15}N_{21}N_5O_3$);
calc: C=56.41, H=6.63, N=21.93,
found: C=54.65, H=6.45, N=19.59.
$^1$H NMR (DMSO-$d_6$, 90 MHz): δ 1.3 (d, 3H), δ 1.3—2.0 (m, 8H), δ 3.9—4.0 (m, 2H), δ 4.3—4.9 (m, 4H), δ 5.8 (d, 1H), δ 7.5—7.6 (d, 1H), δ 8.1 (s, 1H), δ 8.2 (s, 1H).

The starting purine was prepared as follows: a solution of cyclopentylamine (4.3 g, 50 mmol), triethylamine (5.05 g, 50 mmol) and 6-chloropurine (7.73 g, 50 mmol) in ethanol (250 mL) was stirred at reflux, overnight. The solution cooled to 45°C and the ethanol removed in vacuo. The residue dissolved in chloroform and washed once with water. The chloroform was evaporated in vacuo and the residue stirred into water. The precipitated solid was collected by filtration and dried at 45°C in vacuo, overnight; yield 6.7 g (66%): mp=187—191°C.

Anal ($C_{10}H_{13}N_5$):
calc: C=59.09, H=6.45, N=34.46,
found: C=58.75, H=6.18, N=34.37.

Example 6

$N^6$-[Cyclohexyl]-5'-deoxyadenosine

The title compound was prepared as Example 1 using 2.2 g, 10 mmol of $N^6$-cyclohexylpurine and 3.1 g, 12 mmol of the triacetate-5-deoxy sugar, and purified using 10% MeOH:$CHCl_3$ solvent for silica gel chromatography. The hydrolysis used 0.25 g of sodium methoxide and yielded; 0.61 g (18%), an extremely hygroscopic syrup.

Anal ($C_{16}H_{23}N_5O_3 \cdot H_2O$);
calc: C=54.69, H=7.17, N=19.93,
found: C=54.92, H=7.28, N=15.90.
$^1$H NMR (DMSO-$d_6$, 90 DMHz): δ 1.2 (d, 3H), δ 1.1—2.0 (m, 8H), δ 3.2—3.5 (m, 2H), δ 4.5—5.3 (m, 6H), δ 5.7 (d, 1H), δ 7.3—7.5 (d, 1H), δ 8.05 (s, 1H), δ 8.15 (s, 1H).

The starting purine was prepared as the starting material in Example 5 using cyclohexylamine (2.0 g, 20 mmol), triethylamine (2.2 g, 20 mmol) and 6-chloropurine (3.1 g, 20 mmol); yield 3.4 g (79%): mp=199—201°C.

Example 7

(R)-$N^6$[Phenylisopropyl]-5'-deoxy-5'-thiomethyladenosine

The title compound was prepared as in Example 11 using 5.0 g (12.9 mmol) (R)-$N^6$-[phenylisopropyl]adenosine, 13.2 g (65 mmol) tri-n-butyl phosphine, and 6.1 g (65 mmol) dimethyldisulfide, yield 28%.

Anal. Calcd. for: $C_{20}H_{25}N_5O_3S$
calc: C, 57,81; H, 6.06; N, 16.86; S, 7.72;
found: C, 57.71; H, 6.28; N, 16.49; S, 8.07.

Example 8

(S)-$N^6$-[Phenylisopropyl]-5'-deoxy-5'-thiomethyladenosine

The intermediate (S)-$N^6$-[phenylisopropyl]-adenosine-2',3'-isopropylidene was prepared as in Example 12 using (S)-$N^6$-[phenylisopropyl]-adenosine (8.4 g, 22 mmol), dimethoxypropane (25 mL) and Hampton's reagent (8.5 g, 25 mmol). [see J. Am. Chem. Soc. *83,* 3640 (1961)]. Yield 61%; this compound (13.3 mmol) was then treated with thionylchloride (1.8 g, 15 mmol) in the manner described in Example 12; this compound (3.7 g, 8.3 mmol) was then treated with lithium methyl mercaptan (2.3 g, 42 mmol) (yield 53%), and deprotected as in Example 12 to give 81% final product, mp 23—24°C.

Anal. Calcd. for: $C_{20}H_{25}N_5O_3S$
calc: C, 57.81; H, 6.06; N, 16.86; S, 7.72;
found: C, 56.81; H, 5.77; N, 15.83; S, 8.74.
$^1$ NMR (DMSO-$d_6$, 90 MHz): δ 1.2 (d, 3H), δ 2.0 (s, 3H), δ 2.6—3.0 (m, 4H), δ 4.1 (m, 2H), δ 4.6 (m, 2H), δ 5.2 (d, 1H), δ 5.4 (d, 1H), δ 5.85 (d, 1H), δ 7.0—7.3 (m, 5H), δ 7.6 (br.d, 1H), δ 8.15 (s, 1H), δ (s, 1H).

Example 9

N⁶-[Cyclohexyl]-5'-deoxy-5'-thiomethyladenosine

$N^6$-[Cyclohexyl]-5'-deoxy-5'-thiomethyladenosine was prepared in a manner analogous to Example 12 using 5.0 g $N^6$-[cyclohexyl]-5'-deoxy-5'-chloroadenosine-2',3'-isopropylidene and lithium methyl mercaptide (2.7 g) to give the 5'-thiomethyl-isopropylidene intermediate, followed by deprotection to give the final product, yield 58%.

Anal. Calcd. for: $C_{17}H_{25}N_5O_3S$

calc: C, 53.81; H, 6.64; N, 18.46; S, 8.48;

found: C, 53.00; H, 6.66; N, 18.19; S, 6.88; water 2.35.

$^1$H NMR (DMSO-$d_6$, 90 MHz): δ 1.1-2.0 (m, 10H), δ 2.0 (s, 3H), δ 2.8 (m, 1H), δ 3.8—4.3 (m, 4H), δ 4.65 (m, 1H), δ 5.1—5.4 (m, 2H), δ 5.85 (d, 1H), δ 7.4 (br.d, 1H), δ 8.1 (s, 1H), δ 8.25 (s, 1H).

$N^6$-[Cyclohexyl]-5'-deoxy-5'-chloroadenosine-2',3'-isopropylidene was synthesized as in Example 12 utilizing 12.5 g. $N^6$-[Cyclohexyl]adenosine, dimethoxy propane 80 ml and Hampton's catalyst 15.2 g to give the isopropylidene, followed by treatment with 5.3 g thionyl chloride. The compound was isolated as the hydrochloride salt.

Example 10

$N^6$-[Cyclopentyl]-5'-deoxy-5'-thiomethyladenosine

The title compound was prepared using $N^6$-[cyclopentyl]-5'-deoxy-5'-chloroadenosine-2',3'-isopropylidene (5.7 g, 13.2 mmol) and lithium methyl mercaptide (4.3 g, 80 mmol) as in Example 12, yield 74%. This was deprotected as in Example 12 to give $N^6$-[cyclopentyl]-5'-deoxy-5'-thiomethyl adenosine, yield 69%.

Anal. Calcd. for: $C_{16}H_{23}N_5O_3S$

calc: C, 52.59; H, 6.34; N, 19.16; S, 8.77;

found: C, 51.67; H, 6.67; N, 17.25; S, 8.59.

$^1$H NMR (DMSO-$d_6$, 90 MHz): δ 1.4—2.1 (m, 8H), δ 2.0 (s, 3H), δ 2.8 (m, 2H), δ 3.9—4.2 (m, 2H), δ 4.5—4.8 (m, 3H), δ 5.85 (d, 1H), δ 7.6 (br.d, 1H), δ 8.2 (s, 1H), δ 8.3 (s, 1H).

The 5'-deoxy-5'-chloroisopropylidene intermediate was synthesized as in Example 12 using $N^6$-[cyclopentyl]-adenosine (5 g, 15 mmol), dimethoxy propane (18 ml) and Hampton's reagent (6.3 g, 19 mmol), yield 86%; following this treatment with 4.1 g (10 mmol) thionyl chloride isolated the intermediate as the hydrochloride salt (yield 49%).

Example 11

$N^6$-[2,2-Diphenyl]-5'-deoxy-5'-thiomethyladenosine

$N^6$-(2,2-Diphenylethyl)adenosine (4.7 g, 10.5 mmol) and tri-n-butylphosphine (10.8 g, 53 mmol) in DMF (20 mL) was stirred, under $N_2$, at room temperature for 48 hours. The reaction mixture was diluted with water (20 mL) and the solvents were removed by distillation (<90°C, 1 mmHg). The glossy black residue was purified by silica gel chromatography eluting with ethyl acetate. The major fraction was isolated by evaporation of the solvent to give a white foam; yield 1.4 g (28%), mp 70—90°C.

Anal. Calcd. for: $C_{25}H_{27}N_5O_3S$

calc: C, 62.87; H, 5.70; N, 14.66; S, 6.71;

found: C, 62.32; H, 5.73; N, 14.25; S, 6.72.

$^1$H NMR (DMSO-$d_6$, 200 MHz): δ 2.0 (s, 3H), δ 2.8 (m, 2H), δ 4.0—4.2 (m, 4H), δ 4.6 (m, 1H), δ 4.7 (m, 1H), δ 5.3 (d, 1H), δ 5.5 (d, 1H), δ 5.9 (d, 1H), δ 7.1—7.4 (m, 10H), δ 7.8 (br.s, 1H), δ 8.3 (br.s, 2H).

$N^6$-(2,2-Diphenylethyl)adenosine was prepared as follows: In 250 ml of absolute ethanol was refluxed 14.3 g 6-chloropurine riboside, 9.8 g (2,2-diphenyl)ethylamine and 5.0 g of triethylamine for 18 hours. The solution was cooled to 8°C and solids collected by filtration. The solids were partitioned between 100 ml methylene chloride and 100 ml water. The organics were dried over magnesium sulfate and the solvent removed in vacuo to give a foam, dried on high vacuum for four hours, mp 88—101°C.

Anal. Calcd. for: $C_{24}H_{25}N_5O_7 \cdot 0.5H_2O$

calc: C, 63.14; H, 5.74; N, 15.34;

found: C, 62.82; H, 5.71; N, 15.10.

Example 12

$N^6$-[1-Indanyl]-5'-deoxy-5'-thiomethyladenosine

A mixture of $N^6$-[1-indanyl]-5'-deoxy-5'-chloro-2',3'-isopropylidene adenosine (3.3 g, 6.9 mmol) and lithium methyl mercaptide (2.2 g, 40 mmol) in THF (200 mL) was heated to reflux and stirred 72 hours. The reaction cooled to room temperature and quenched with water (100 mL). The THF was removed in vacuo and the solution extracted with methylene chloride (100 mL). The organics were dried over $MgSO_4$ and the solvent was removed in vacuo to give a foam. The foam, the 5'-SMe-5'-deoxy-isopropylidene derivative, was dried on high vacuum at room temperature, 4 hours; yield 1.2 g (39%). This material was stirred into 50% aqueous formic acid (100 mL) and stirred at 50°C for four hours. The formic acid/water was removed in vacuo and the residue coevaporated to dryness with methanol, to give a foam. The foam was purified by silica gel chromatography eluting with acetone, and the major component was isolated by evaporation of

the solvent. The resultant hygroscopic yellow foam was dried on high vacuum at room temperature; yield 1.3 g (81%).

Anal. Calcd. for: $C_{20}H_{23}N_5O_3SH_20$

calc: C, 55.67; H, 5.61; N, 16.23; S, 7.41;

found: C, 56.41; H, 5.91; N, 15.17; S, 5.74.

$^1$H NMR (DMSO-$d_6$, 200 MHz): δ 2.0 (s, 3H), δ 2.8—3.1 (m, 5H), δ 4.0—4.2 (m, 2H), δ 4.5 (m, 1H), δ 4.8 (m, 1H), δ 5.3 (d, 1H), δ 5.5 (d, 1H), δ 5.9 (d, 1H), δ 5.9—6.0 (br.s, 1H), δ 7.1—7.3 (m, 4H), δ 8.1 (br.d, 1H), δ 8.3 (br.s, 1H), δ 8.5 (s, 1H).

The starting material was prepared as follows: A solution of dimethoxy propane (30 mL), $N^6$-(1-indanyl)-adenosine (9.0 g, 23.5 mmol) and Hampton's reagent (bis-p-nitrophenylphosphate hydrate), [J. Am. Chem. Soc. *83,* 3640 (1961)], (9.6 g, 28 mmol) in acetone (150 mL) was stirred at room temperature, with exclusion of moisture, overnight. The reaction was quenched with ~5% $NaHCO_3$ solution (125 mL) and the solution stirred for one half hour. The acetone was removed in vacuo and the residue dissolved in methanol. The methanolic solution was poured through a Dowex 1 × 8 ($NH_4^+HCO_3^-$) column, eluted with methanol (300 mL). The methanol was removed in vacuo to give the isopropylidene, yield 6.4 g (65%). The isopropylidene (6.1 g, 14.4 mmol) was placed in THF (150 mL) and treated with thionyl chloride (2.4 g, 20 mmol). The solution was warmed to reflux for one hour then cooled to room temperature and stirred overnight. The THF was removed in vacuo at 40°C and the residue dissolved in methylene chloride (150 mL). The solution was washed with water (2 × 100 mL) and dried over $MgSO_4$. The solvent was then removed in vacuo to give a yellow foam, the $N^6$-(indanyl)-5′-chloro-5′-deoxy-isopropylidene adenosine 0.8 HCl, yield 5.5 g (81%).

$N^6$-(1-Indanyl)adenosine was in turn prepared as follows: Four grams 6-chloropurine riboside, 2.32 g 1-aminoindane and 2.11 g of triethylamine were refluxed in 100 ml of absolute ethanol under nitrogen for 20 hours. Volatiles were evaporated to dryness. Residue was dissolved in 20 ml 2-propanol and diluted with 200 ml $H_2O$. Clear aqueous solution was decanted and residual oily material washed with cold water. Coevaporation with ethanol several times gave solid material. Crystallization from $CHCl_3$/2-propanol (10:1) and hexane afforded 3.0 g (56.2%) $N^6$-(1-indanyl)adenosine having a melting point of 120—122°C (foam).

Anal. Calcd. for: $C_{19}H_{21}N_5O_4$

calc: C, 59.52; H, 5.52; N, 18.26;

found: C, 59.37; H, 5.48; N, 18.00.

Example 13

$N^6$-[2,2-Diphenylethyl]-5′-deoxy-5′-chloroadenosine

The intermediate $N^6$-[2,2-diphenylethyl]-5′-deoxy-5′-chloroadenosine-2′,3′-isopropylidene was synthesized as in Example 14 from $N^6$-[2,2-diphenylethyl]adenosine (2.2 g, 4.7 mmol), dimethoxypropane (8 ml) and Hampton's reagent* (1.9 g, 5.6 mmol), (yield 1.8 g, 78%), followed by treatment with 1.5 equivalents of thionylchloride (yield 59%). The isopropylidene was hydrolyzed to the final compound as in Example 14 (yield 36%) mp 180—183°C.

Analysis as ($C_{24}H_{24}ClN_5O_3$)

calc'd: C, 61.87; H, 5.19; N, 15.03; Cl, 7.61;

found: C, 61.48; H, 5.19; N, 14.62; Cl, 8.51.

$^1$H NMR (DMSO-$d_6$, 200 MHz): δ 3.8—3.9 (m, 2H), δ 4.0—4.2 (m, 3H), δ 4.6 (m, 2H), δ 4.7 (d of d, 1H), δ 5.4 (d, 1H), δ 5.55 (d, 1H), δ 5.9 (d, 1H), δ 7.1—7.3 (m, 10H), δ 7.8 (br.s, 1H), δ 8.2 (s, 2H).

The starting $N^6$-(2,2-diphenylethyl)adenosine was prepared as follows:

In 250 ml of absolute ethanol was refluxed 14.3 g 6-chloropurine riboside, 9.8 g (2,2-diphenyl)ethylamine and 5.0 g of triethylamine for 18 hours. The solution cooled to 8°C and solids collected by filtration. The solids were partitioned between 100 ml methylene chloride and 100 ml water. The organics was dried over magnesium sulfate and the solvent removed in vacuo to give a foam dried on high vacuum for four hours. mp 88—101°C.

Anal ($C_{24}H_{25}N_5O_7\cdot 0.5H_2O$)

calc: C, 63.14; H, 5.74; N, 15.34;

found: C, 62.82; H, 5.71; N, 15.10.

Example 14

$N^6$-[1-Indanyl]-5′-deoxy-5′-chloroadenosine

A solution of dimethoxy propane (30 ml), $N^6$-(1-indanyl)adenosine (9.0 g, 23.5 mmol) and Hampton's reagent* (bis-p-nitrophenylphosphate hydrate) (9.6 g, 28 mmol) in acetone (150 ml) was stirred at room temperature, with exclusion of moisture, overnight. The reaction was quenched with ~5% $NaHCO_3$ solution (125 mL) and the solution stirred for one half hour. The acetone was removed in vacuo and the residue dissolved in methanol. The methanolic solution was poured through a Dowex 1 × 8 ($NH_4^+HCO_3^-$) column, eluted with methanol (300 mL). The methanol was removed in vacuo to give the isopropylidene,

* J. Am. Chem. Soc., *83,* 3640 (1961)

yield 6.4 g (65%). The isopropylidene (6.1 g, 14.4 mmol) was placed in THF (150 mL) and treated with thionyl chloride (2.4 g, 20 mmol). The solution was warmed to reflux for one hour then cooled to room temperature and stirred overnight. The THF was removed in vacuo at 40°C and the residue dissolved in methylene chloride (150 mL). The solution was washed with water (2 × 100 mL) and dried over $MgSO_4$. The solvent was then removed in vacuo to give a yellow foam, the 5'-chloro-5'-deoxyisopropylidene·0.8HCl, yield 5.5 g (81%). The protected 5'-chloro compound (2.0 g, 4.2 mmol) was then dissolved in 50% aqueous formic acid (110 mL) and the solution was warmed to 60°C for four hours. The formic acid/water was removed in vacuo and the residue coevaporated to dryness with methanol to give a white foam. This foam was purified by silica gel chromatography eluting with acetone. The major component was isolated by evaporation of acetone to give a very hygroscopic white solid, dried in vacuo at room temperature, overnight; yield 0.85 g (50%).

Anal ($C_{19}H_{20}ClN_5O_3 \cdot H_2O$)
calc: C, 54.35; H, 5.04; N, 16.68; Cl, 8.44;
found: C, 54.52; H, 5.40; N, 15.47; Cl, 8.54.

$^1$H NMR (DMSO-$d_6$, 100 MHz): δ 1.0—1.5 (m, 2H), δ 2.8—3.1 (m, 2H), δ 3.9 (d of d, 2H), δ 4.0—4.3 (m, 2H), δ 4.8 (d of d, 1H), δ 5.5 (d, 1H), δ 5.6 (d, 1H), δ 5.95 (d, 1H), δ 7.1—7.3 (m, 4H), δ 8.2 (br.d, 1H), δ 8.3 (s, 1H), δ 8.35 (s, 1H).

The starting N(6)-(1-Indanyl)adenosine was prepared as follows:

Four grams 6-chloropurine riboside, 2.32 g 1-aminoindane and 2.11 g of triethylamine were refluxed in 100 ml of absolute ethanol under nitrogen for 20 hours. Volatiles were removed. The residue was dissolved in 20 ml 2-propanol and diluted with 200 ml $H_2O$. Clear aqueous solution was decanted and residual oily material washed with cold water. Coevaporation with ethanol several times gave solid material. Crystallization from $CHCl_3$/2-propanol (10:1) and hexane afforded 3.0 g (56.2%) N(6)-(1-indanyl)adenosine having a melting point of 120—122°C (foam).

Anal ($C_{19}H_{21}N_5O_4$)
calc: C, 59.52; H, 5.52; N, 18.26;
found: C, 59.37; H, 5.48; N, 18.00.

Example 15

(R)-N$^6$-[Phenylisopropyl]-5'-deoxy-5'-chloro-adenosine

A solution of thionylchloride (8.3 g, 70 mmol) in HMPA (25 mL) was treated with (R)-N$^6$-(phenylisopropyl)adenosine (5 g, 13 mmol) and the mixture stirred at room temperature, overnight. The reaction was quenched with water (80 mL) and neutralized within ammonium hydroxide. The gum that formed was separated from the solution, and washed with water (30 mL). The gum was dissolved in ethanol and evaporation in vacuo to give a foam. The compound was dissolved in ethanol (20 mL) and passed through silica gel, eluted with ethanol (100 mL). The ethanol evaporated in vacuo. The residue was dissolved in methylene chloride and dried over $MgSO_4$ evaporated in vacuo to give a yellow foam; yield 1.65 g (31%): mp=83—86.

Anal ($C_{19}H_{22}ClN_5O_3$)
calc: C, 56.50; H, 5.49; N, 17.34; Cl, 8.78;
found: C, 56.34; H, 5.60; N, 17.37; Cl, 8.41.

Example 16

(S)-N$^6$-[Phenylisopropyl]-5'-deoxy-5'-chloroadenosine

The title compound was synthesized as in Example 15 using (S)-N$^6$-(phenylisopropyl)adenosine (15.4 g, 40 mmol), HMPA (80 mL) and thionyl chloride (25.5 g, 215 mmol); yield 11% mp 75—110°.

Analysis for ($C_{19}H_{22}ClN_5O_3$)
calc: C, 56.60; H, 5.49; N, 17.34; Cl, 8.78;
found: C, 56.14; H, 5.42; N, 16.99; Cl, 8.77.

$^1$H NMR (DMSO-$d_6$, 90 MHz): δ 1.2 (d, 3H), δ 2.7—3.2 (m, 2H), δ 3.9—4.0 (m, 2H), δ 4.05—4.4 (m, 2H), δ 4.8 (d of d, 2H), δ 5.5 (d, 1H), δ 5.6 (d, 1H), δ 6.0 (d, 1H), δ 7.1—7.4 (m, 5H), δ 7.7 (br.d, 1H), δ 8.2 (s, 1H), δ 8.3 (s, 1H).

Example 17

N$^6$-[Cyclohexyl]-5'-deoxy-5'-chloro-adenosine

N$^6$-[Cyclohexyl]-5'-deoxy-5'-chloro-adenosine was synthesized as in Example 14 utilizing 12.5 g, N$^6$-[cyclohexyl]adenosine, dimethoxypropane 80 mL and Hampton's catalyst 15.2 g to give the isopropylidene, followed by treatment with 5.3 g, thionyl chloride and deprotection. Yield 40% mp 65—85°.

Analysis for ($C_{16}H_{22}N_5O_3Cl$)
calc: C, 52.24; H, 6.03; N, 19.04; Cl, 9.64;
found: C, 51.20; H, 5.72; N, 17.26; Cl, 13.32.

$^1$H NMR (DMSO-$d_6$, 90 MHz): δ 1.1—2.0 (m, 11H), δ 3.8—4.0 (m, 2H), δ 4.0—4.3 (m, 2H), δ 4.75 (d of d, 1H), δ 5.45 (d, 1H), δ 5.55 (d, 1H), δ 5.9 (d, 1H), δ 7.5 (br.d, 1H), δ 8.2 (s, 1H), δ 8.3 (s, 1H).

Example 18

$N^6$-[Cyclopentyl]-5'-deoxy-5'-chloroadenosine

The isopropylidene intermediate was synthesized as in Example 14 using $N^6$-[cyclopentyl]-adenosine (5 g, 15 mmol), dimethoxypropane (18 ml) and Hampton's reagent (6.3 g, 19 mmol) yield 86%. The 5'-chloro-5'-deoxy isopropylidene analog was synthesized as in Example 14 using 4.1 g (10 mmol) $N^6$-[cyclopentyl]-adenosine-2'-3'-isopropylidene and 1.3 g (10 mmol) thionyl chloride and was isolated as the hydrochloride salt (yield 49%). This was hydrolyzed as in Example 14 to give $N^6$-[cyclopentyl]-5'-deoxy-5'-chloroadenosine. Mp ~70°C.

Anal ($C_{15}H_{20}ClN_5O_3$)

| | | | | |
|---|---|---|---|---|
| calc: | C, 50.92; | H, 5.70; | N, 19.80; | Cl, 10.02; |
| found: | C, 50.54; | H, 5.84; | N, 19.12; | Cl, 10.64. |

$^1H$ NMR (DMSO-$d_6$, 90 MHz): δ 1.4—2.1 (m, 9H), δ 3.85 (m, 3H), δ 4.2 (m, 2H), δ 4.6 (m, 2H), δ 5.9 (d, 1H), δ 8.4 (s, 1H), δ (s, 1H), δ 8.6 (s, 1H), δ 9.5 (s, 1H).

Example 19

$N^6$-1-Tetralinyl-5'-deoxyadenosine

Six chloropurine (7.7 g, 50 mmol), triethyl amine (5.1 g, 50 mmol) and 1-aminotetralin (7.4 g, 50 mmol) were stirred at reflux in 150 ml of ethanol, overnight. The solution was cooled to 0°C and filtered free of precipitate. The precipitate washed with 50 ml of cold (0°C) ethanol and dried at 60°C in vacuo for four hours to give 4.2 g (32%) of a white solid, mp 232.5—234°C.

Anal $C_{15}H_{15}N_5$

| | | | |
|---|---|---|---|
| calc: | C, 67.90; | H, 5.70; | N, 26.40; |
| found: | C, 67.99; | H, 5.62; | N, 26.41. |

The adenine (5.3 g, 20 mmol) and 5'-deoxy ribose triacetate (6.5 g, 25 mmol) were melted and stirred at 190°C. To the melt was added one micro drop of concentrated sulfuric acid and the acetic acid formed was removed in a gentle stream of nitrogen. The solution was stirred for two hours before cooling to room temperature. The glassy residue was broken up in 20 ml of ethyl acetate in an ultrasonic bath. The solution was then purified by chromatography to give after evaporation of the solvent 4.7 g of a light brown foam identified herein after as the compound D in Example 22. The foam (4.0 g, 8.6 mmol) was then dissolved in 100 ml of methanol and sodium methoxide (0.5 g, 8.6 mmol) added. The solution was stirred at room temperature for four hours. The reaction was neutralized with Dowex 50 × 8 (H+ form) to pH 7 and the resin removed by filtration. The filtrate was evaporated in vacuo and the residue stirred into 4.0 ml of acetone and the solid collected by filtration and dried at 40°C overnight, to give 1.95 g (30%) of a hydroscopic white solid, mp 159—162°C.

Anal $C_{20}H_{23}N_5O_3$

| | | | |
|---|---|---|---|
| calc: | C, 62.98; | H, 6.08; | N, 18.36; |
| found: | C, 62.87; | H, 6.21; | N, 18.18. |

Example 20

$N^6$-α-Naphthylmethyl-5'-deoxyadenosine

Six chloropurine (7.6 g, 50 mmol), triethyl amine (5.1 g, 50 mmol) and α-naphthylmethyl amine (7.9 g, 50 mmol) were stirred, at reflux, in 150 ml of ethanol, overnight. The solution was cooled to 0°C and filtered free of precipitate and the precipitated solid washed with cold ethanol. The solid was dried at room temperature in vacuo overnight to give 10.6 g (77%) of a white solid, mp 250—252.5°C.

The adenine (5.5 g, 20 mmol) and 5' deoxy ribose (6.5 g, 25 mmol) were melted and stirred at 200—220°C. To the melt was added one micro drop of concentrated sulfuric acid and the acetic acid formed was removed in a gentle stream of nitrogen. The solution was stirred for six hours before cooling to room temperature. The glassy residue was broken up in 30 ml of ethyl acetate in an ultrasonic bath. The solution was then purified by chromatography to give after evaporation of the solvent 3.9 g of a white foam that is the compound identified as 21 hereinafter. The foam (3.0 g, 6.3 mmol) was then dissolved in 100 ml of methanol and sodium methoxide (0.4 g, 6.3 mmol) added. The solution was stirred at room temperature for four hours. The reaction was neutralized with Dowex 50 × 8 (H+ form) to pH 7 and the resin removed by filtration. The filtrate was evaporated in vacuo and the residue recrystallized from 30 ml of ethanol. The solid was collected by filtration and dried at 40°C in vacuo, overnight, to give 1.9 g (31%) of a hygroscopic light brown solid, mp 124—128°C.

Example 21

$N^6$-α-Naphthylmethyl-2',3'-di-O-acetyl-5'-deoxyadenosine

Compound 21 from Example 20 was analyzed to give the product $N^6$-(α-naphthyl)methyl-5'-deoxy-2',3'-diacetyl adenosine 3.9 g (41%), mp 70—78°C.

Anal $C_{25}H_{25}N_5O_5$

| | | | |
|---|---|---|---|
| calc: | C, 63.15; | H, 5.30; | N, 14.73; |
| found: | C, 62.91; | H, 5.50; | N, 14.57. |

Example 22

$N^6$-1-Tetralinyl-2',3'-di-O-acetyl-5'-deoxyadenosine

Compound D from Example 19 was analyzed to give the product $N^6$-(1-tetralinyl)-5'-deoxy-2',3'-diacetyladenosine 4.7 g (51%), mp 70—72°C.

Anal $C_{24}H_{27}N_5O_5$

calc: C, 61.92; H, 5.85; N, 15.05;

found: C, 61.98; H, 6.05; N, 14.96.

Example 23

(R)-$N^6$(1-Indanyl)-5'-deoxy-5'-chloroadenosine

A solution of dimethoxy propane (25 ml), $N^6$-(R)-1-indanyl-adenosine (9.6 g, 25 mmol) and bis-p-nitrophenyl-phosphate hydrate (9.5 g, 28 mmol) in acetone (200 ml) was stirred at room temperature, overnight. The solution was quenched with water (150 ml) and the acetone evaporated in vacuo. The aqueous solution was extracted with methylene chloride (2 × 150 ml) and the organic solution washed with water (100 ml) and then saturated salt solution (100 ml) and then saturated salt solution (100 ml). The organic solution was dried over magnesium sulfate and the solvent evaporated in vacuo. The residue was dissolved in methanol (75 ml) and passed through a short column of Dowex 1 × 8 (sodium bicarbonate form) ion exchange resin. The column washed with methanol (580 ml total). The methanol was then evaporated in vacuo to give the isopropylidene analog 8.8 g (83%). The isopropylidene (7.5 g, 17.7 mmol) was placed in THF (100 ml), treated with thionyl chloride (3.2 g, 26.5 mmol) and stirred at room temperature overnight. The solution was evaporated in vacuo to dryness and the residue dissolved in methylene chloride (100 ml). The organic solution was washed with water (100 ml), saturated salt solution (100 ml) and dried with magnesium sulfate. The solvents were evaporated in vacuo and the residue purified by chromatography to give, after evaporation of solvent from the major RI active fraction, 5.5 g (71%) of a light yellow foam. The foam (4.8 g, 10.9 mmol) was then dissolved in 50% formic acid (100 ml) and stirred at 50°C for 4.5 hours. The solution was evaporated in vacuo to dryness and the residue purified by chromatography. This gave, after evaporation of solvent from the major RI active fraction, 2.5 g (57%) of a white foam, mp 94—97°C.

Anal ($C_{19}H_{20}ClN_5O_3$)

calc: C, 56.79; H, 5.02; N, 17.43;

found: C, 56.60; H, 5.18; N, 17.60.

**Claims for the Contracting States: BE CH DE FR GB IT LI LU NL SE**

1. A compound of the following general formula (I)

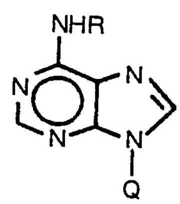

(I)

or a pharmaceutically acceptable acid addition salt thereof, wherein:

R is cycloalkyl having from three to eleven ring members or R is one of the following groups of the formulae

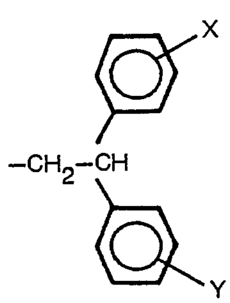 , 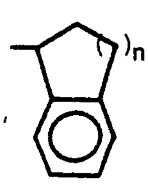 , 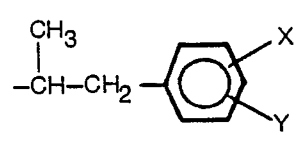 or 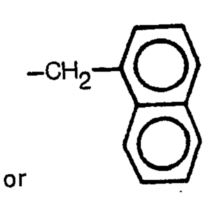

(Ia) (Ib) (Ic) (Ie)

in which

n is one, two, or three;

X and Y are each, independently, H, $C_1$—$C_6$ alkyl, hydroxy, $C_1$—$C_6$ alkoxy, benzyloxy, nitro, amino or halogen; and

Q is of the formula:

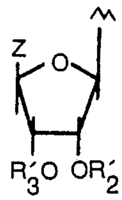

in which

Z is $CH_3$, $CH_2Hal$ in which Hal stands for a halogen atom or $CH_2SCH_3$; and

$R_2'$ or $R_3'$ are each, independently, H, $C_1$—$C_6$ alkyl, $C_2$—$C_7$ alkanoyl, benzoyl, benzoyl substituted by $C_1$—$C_6$ alkyl, $C_1$—$C_6$ alkoxy, or halogen or when taken together are $C_1$—$C_6$ alkylidene where the hydroxy groups are free or $C_2$—$C_7$ alkanoyl or benzoyl esters thereof.

2. A compound according to Claim 1, wherein Z is —$CH_3$.

3. A compound according to Claim 1, wherein Z is —$CH_2SCH_3$.

4. A compound according to Claim 1, wherein Z is —$CH_2Hal$ in which Hal is a halogen atom, preferably chlorine.

5. A compound according to Claim 2, 3 or 4, wherein R is of the formula (Ia):

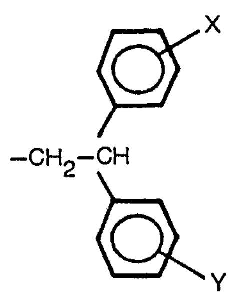

(Ia)

6. A compound according to Claim 2, 3 or 4, wherein R is of the formula (Ib):

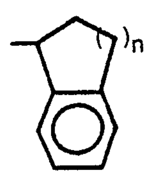

(Ib)

7. A compound according to Claim 2, 3 or 4, wherein R is of the formula (Ic):

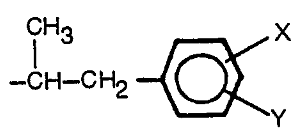

(Ic)

8. A compound according to Claim 2, 3 or 4, wherein R is of the formula (Ie):

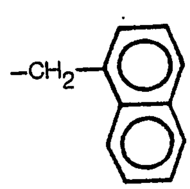

(Ie)

9. A compound according to Claim 2, 3 or 4, wherein R is a cycloalkyl radical having from three to eleven ring members, preferably from five to seven ring members.

10. A compound according to Claim 5, 6 or 7, wherein X and Y are hydrogen.

11. A compound according to Claim 10, wherein Q is 5'-deoxy-β-D-ribose and $OR_2'$ and $OR_3'$ are hydroxy or acetyl esters thereof.

12. A compound according to Claim 11 when appendant to Claims 2 and 5, and being 5'-deoxy-$N^6$-(2,2-diphenylethyl)adenosine.

13. A compound according to Claim 11 when appendant to Claims 2 and 6, and being chosen from: 5'-deoxy-$N^6$-(1-indanyl)adenosine; $N^6$-1-tetralinyl-5'-deoxyadenosine; and $N^6$-1-tetralinyl-5'-deoxyadenosine-2',3'-di-O-acetyl.

14. A compound according to Claim 11 when appendant to Claims 2 and 7, and being chosen from: (R)-5'-deoxy-$N^6$-(1-methyl-2-phenylethyl) adenosine; and (S)-5'-deoxy-$N^6$-(1-methyl-2-phenylethyl)adenosine.

15. A compound according to Claim 8 when appendant to Claim 2, and being chosen from: $N^6$-α-naphthylmethyl-5'-deoxyadenosine; and $N^6$-α-naphthylmethyl-3'-deoxyadenosine-2',3'-di-O-acetyl.

16. A compound according to Claim 9 when appendant to Claim 2, wherein Q is 5'-deoxy-β-D-ribose and $OR_2'$ and $OR_3'$ are each hydroxy or acetyloxy.

17. A compound according to Claim 16, and being 5'-deoxy-$N^6$-(cyclopentyl)adenosine.

18. A compound according to Claim 10, when appendant to Claim 3, wherein $R_2'$ and $R_3'$ are each independently hydrogen, acetyl, benzoyl or, when taken together, isopropylidene.

19. A compound according to Claim 18, when further appendant to Claim 5, and being: 5'-deoxy-5'-methylthio-$N^6$-(2,2-diphenylethyl)adenosine.

20. A compound according to Claim 18 when further appendant to Claim 6, and being: 5'-deoxy-5'-methylthio-$N^6$-(1-indanyl)adenosine.

21. A compound according to Claim 18 when further appendant to Claim 7, and being chosen from: (R)-5'-deoxy-5'-methylthio-$N^6$-(1-methyl-2-phenylethyl)-adenosine; and (S)-5'-deoxy-5'-methylthio-$N^6$-(1-methyl-2-phenylethyl)-adenosine.

22. A compound according to Claim 9 when appendant to Claim 3, wherein $R_2'$ and $R_3'$ are each independently hydrogen, acetyl, benzoyl or, when taken together, isopropylidene.

23. A compound according to Claim 22, wherein $R_2'$ and $R_3'$ are hydrogen.

24. A compound according to Claim 23 and being: 5'-deoxy-5'-methylthio-$N^6$-(cyclopentyl)adenosine.

25. A compound according to Claim 10 when appendant to Claim 4, wherein $R_2'$ and $R_3'$ are each independently hydrogen, acetyl or benzoyl, or $R_2'$ and $R_3'$ when taken together are isopropylidene.

26. A compound according to Claim 25 when further appendant to Claim 5 and being: 5'-deoxy-5'-chloro-$N^6$-(2,2-diphenylethyl)adenosine.

27. A compound according to Claim 25 when further appendant to Claim 6, and being chosen from: 5'-deoxy-5'-chloro-$N^6$-(1-indanyl)adenosine; and (R)-N-6-(1-indanyl)-5'-deoxy-5'-chloro-adenosine.

28. A compound according to Claim 25 when further appendant to Claim 7, and being chosen from: (R)-5'-deoxy-5'-chloro-$N^6$-(1-methyl-2-phenylethyl)-adenosine; and (S)-5'-deoxy-5'-chloro-$N^6$-(1-methyl-2-phenylethyl)-adenosine.

29. A compound according to Claim 9 when appendant to Claim 4, wherein $R_2'$ and $R_3'$ are each independently hydrogen, acetyl, benzoyl or when taken together an isopropylidene.

30. A compound according to Claim 29, wherein $R_2'$ and $R_3'$ are each hydrogen.

31. A.compound according to Claim 30 and being: 5'-deoxy-5'-chloro-$N^6$-(cyclopentyl)adenosine.

32. A pharmaceutical composition comprising a therapeutically effective amount of a compound as claimed in any preceding claim together with a pharmaceutically acceptable carrier or diluent.

33. For use in a method of treating psychoses in a mammal suffering therefrom, a compound as claimed in any one of Claims 1 to 31, preferably in unit dosage form.

34. For use in a method of treating hypertension in a mammal suffering therefrom a compound as claimed in any one of Claims 1 to 31, preferably in unit dosage form.

35. For use in a method of treating pain in a mammal suffering therefrom, a compound as claimed in any one of Claims 1 to 31, preferably in unit dosage form.

36. A process for the preparation of a compound as claimed in Claim 1, which comprises reacting a N-6-substituted purine of the formula

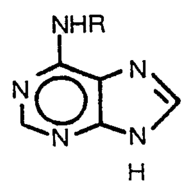

wherein R is as defined in Claim 1, with a compound of the formula

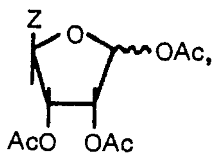

wherein Z is as defined in Claim 1, at elevated for temperatures from about one to about 12 hours and, if desired, converting the resulting free base to a pharmaceutically acceptable acid addition salt thereof.

**Claims for the Contracting State: AT**

1. A process for preparing a compound of the following general formula (I)

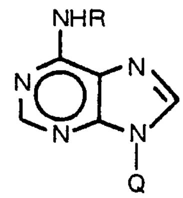

(I)

or a pharmaceutically acceptable acid addition salt thereof, wherein

R is a cycloalkyl having from three to eleven ring members or R is one of the following groups of the formulae

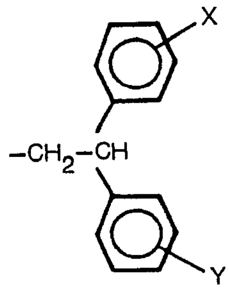

,

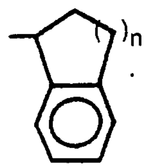

,

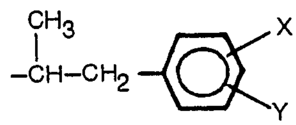

or

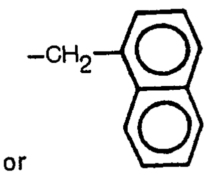

(Ia) (Ib) (Ic) (Ie)

in which

n is one, two, or three;

X and Y are each, independently, H, $C_1$—$C_6$ alkyl, hydroxy, $C_1$—$C_6$ alkoxy, benzyloxy, nitro, amino or halogen; and

Q is of the formula:

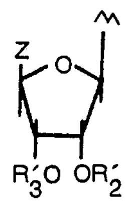

in which

Z is $CH_3$, $CH_2Hal$ in which Hal stands for a halogen atom or $CH_2SCH_3$; and

$R_2'$ or $R_3'$ are each, independently, H, $C_1$—$C_6$ alkyl, $C_2$—$C_7$ alkanoyl, benzoyl, benzoyl substituted by $C_1$—$C_6$ alkyl, $C_1$—$C_6$ alkoxy, or halogen or when taken together are $C_1$—$C_6$ alkylidene where the hydroxy groups are free or $C_2$—$C_7$ alkanoyl or benzoyl esters thereof;

which process comprises reacting an N-6-substituted purine of the formula

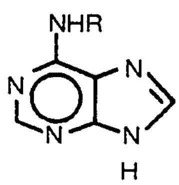

wherein R is as defined above, with a compound of the formula

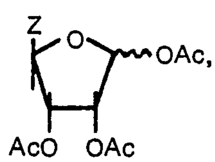

wherein Z is as defined above, at elevated temperatures for from about one to about 12 hours and, if desired, converting the resulting free base to a pharmaceutically acceptable acid addition salt thereof.

2. A process according to Claim 1, wherein Z is —$CH_3$.

3. A process according to Claim 1, wherein Z is —$CH_2SCH_3$.

4. A process according to Claim 1, wherein Z is —$CH_2Hal$ in which Hal is a halogen atom, preferably chlorine.

5. A process according to Claim 2, 3 or 4, wherein R is of the formula (Ia):

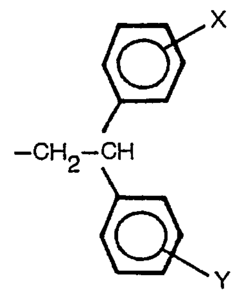

(Ia)

6. A process according to Claim 2, 3 or 4, wherein R is of the formula (Ib):

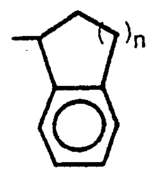

(Ib)

7. A process according to Claim 2, 3 or 4, wherein R is of the formula (Ic):

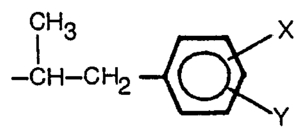

(Ic)

8. A process according to Claim 2, 3 or 4, wherein R is of the formula (Ie):

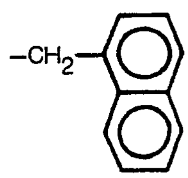

(Ie)

9. A process according to Claim 2, 3 or 4, wherein R is a cycloalkyl radical having from three to eleven ring members, preferably from five to seven ring members.

10. A process according to Claim 5, 6 or 7, wherein X and Y are hydrogen.

11. A process according to Claim 10, wherein Q is 5′-deoxy-β-D-ribose and $OR_2'$ and $OR_3'$ are hydroxy or acetyl esters thereof.

12. A process according to Claim 11 when appendant to Claims 2 and 5, in which a compound having the following name is prepared: 5′-deoxy-$N^6$-(2,2-diphenylethyl)adenosine.

13. A process according to Claim 11 when appendant to Claims 2 and 6, in which a compound having one of the following names is prepared: 5′-deoxy-$N^6$-(1-indanyl)adenosine; $N^6$-1-tetralinyl-5′-deoxyadenosine; and $N^6$-1-tetralinyl-5′-deoxyadenosine-2′,3′-di-O-acetyl.

14. A process according to Claim 11 when appendant to Claims 2 and 7, in which a compound having one of the following names is prepared: (R)-5′-deoxy-$N^6$-(1-methyl-2-phenylethyl)adenosine; and (S)-5′-deoxy-$N^6$-(1-methyl-2-phenylethyl)adenosine.

15. A process according to Claim 8 when appendant to Claim 2, in which a compound having one of the following names is prepared: $N^6$-α-naphthylmethyl-5′-deoxyadenosine; and $N^6$-α-naphthylmethyl-3′-deoxyadenosine-2′,3′-di-O-acetyl.

16. A process according to Claim 9 when appendant to Claim 2, wherein Q is 5′-deoxy-β-D-ribose and $OR_2'$ and $OR_3'$ are each hydroxy or acetyloxy.

17. A process according to Claim 16, in which the following compound is prepared: 5′-deoxy-$N^6$-(cyclopentyl)adenosine.

18. A process according to Claim 10, when appendant to Claim 3, wherein $R_2'$ and $R_3'$ are each independently hydrogen, acetyl, benzoyl or, when taken together, isopropylidene.

19. A process according to Claim 18, when further appendant to Claim 5, in which the following compound is prepared: 5′-deoxy-5′-methylthio-$N^6$-(2,2-diphenylethyl)adenosine.

20. A process according to Claim 18 when further appendant to Claim 6, in which the following compound is prepared: 5′-deoxy-5′-methylthio-$N^6$-(1-indanyl)adenosine.

21. A process according to Claim 18 when further appendant to Claim 7, in which one of the following compounds is prepared: (R)-5'-deoxy-5'-methylthio-$N^6$-(1-methyl-2-phenylethyl)adenosine; and (S)-5'-deoxy-5'-methylthio-$N^6$-(1-methyl-2-phenylethyl)adenosine.

22. A process according to Claim 9 when appendant to Claim 3, wherein $R_2'$ and $R_3'$ are each independently hydrogen, acetyl, benzoyl or, when taken together, isopropylidene.

23. A process according to Claim 22, wherein $R_2'$ and $R_3'$ are hydrogen.

24. A process according to Claim 23 in which a compound having the following formula is prepared: 5'-deoxy-5'-methylthio-$N^6$-(cyclopentyl)adenosine.

25. A process according to Claim 10 when appendant to Claim 4, wherein $R_2'$ and $R_3'$ are each independently hydrogen, acetyl or benzoyl, or $R_2'$ and $R_3'$ when taken together are isopropylidene.

26. A process according to Claim 25 when further appendant to Claims 5, in which a compound having the following formula is prepared: 5'-deoxy-5'-chloro-$N^6$-(2,2-diphenylethyl)adenosine.

27. A process according to Claim 25 when further appendant to Claim 6, in which one of the following compounds is prepared: (5'-deoxy-5'-chloro-$N^6$-(1-indanyl)adenosine; and (R)-N-6-(1-indanyl)-5'-deoxy-5'-chloro-adenosine.

28. A process according to Claim 25 when further appendant to Claim 7 in which one of the following compounds is prepared: (R)-5'-deoxy-5'-chloro-$N^6$-(1-methyl-2-phenylethyl)-adenosine; and (S)-5'-deoxy-5'-chloro-$N^6$-(1-methyl-2-phenylethyl)-adenosine.

29. A process according to Claim 9 when appendant to Claim 4, wherein $R_2'$ and $R_3'$ are each independently hydrogen, acetyl, benzoyl or when taken together an isopropylidene.

30. A process according to Claim 29, wherein $R_2'$ and $R_3'$ are each hydrogen.

31. A process according to Claim 30, in which the following compound is prepared: 5'-deoxy-5'-chloro-$N^6$-(cyclopentyl)adenosine.

32. A process for preparing a pharmaceutical composition comprising combining a therapeutically effective amount of a compound prepared by a process as claimed in any preceding claim together with a pharmaceutically acceptable carrier or diluent.

33. For use in a method of treating psychoses in a mammal suffering therefrom, a compound prepared by a process as claimed in any one of Claims 1 to 31, preferably in unit dosage form.

34. For use in a method of treating hypertension in a mammal suffering therefrom, a compound prepared by a process as claimed in any one of Claims 1 to 31, preferably in unit dosage form.

35. For use in a method of treating pain in a mammal suffering therefrom, a compound prepared by a process as claimed in any one of Claims 1 to 31, preferably in unit dosage form.

**Patentansprüche für die Vertragsstaaten: BE CH DE FR GB IT LI LU NL SE**

1. Verdindung der folgenden allgemeinen Formel (I)

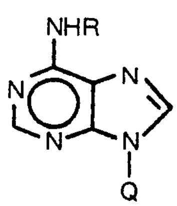

(I)

oder ein pharmazeutisch akzeptables Säureadditionssalz davon, worin

R Cycloalkyl mit 3 bits II Ringgliedern ist oder R einr der folgenden Gruppen der Formeln

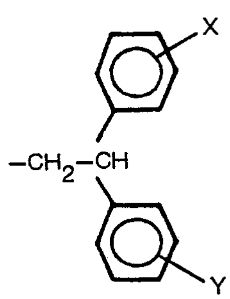 ,

(Ia)

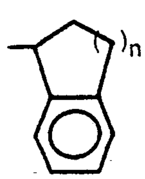 ,

(Ib)

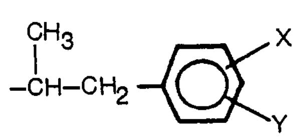

(Ic)

oder

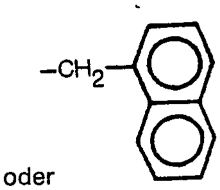

(Ie)

worin

n für 1, 2 oder 3 steht;

X und Y jeweils unabhängig voneinander H, $C_1$—$C_6$-Alkyl, Hydroxy, $C_1$—$C_6$-Alkoxy, Benzyloxy, Nitro, Amino oder Halogen bedeuten, ist, und

Q die Formel

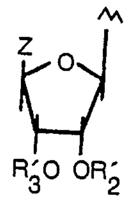

darstellt, worin

Z für $CH_3$, $CH_2Hal$, worin Hal ein Halogenatom oder $CH_2SCH_3$ bedeutet, steht; und

$R_2'$ oder $R_3'$ jeweils unabhängig voneinander H, $C_1$—$C_6$-Alkyl, $C_2$—$C_7$-Alkanoyl, Benzoyl, Benzoyl substituiert durch $C_1$—$C_6$-Alkyl, $C_1$—$C_6$-Alkoxy oder Halogen bedeuten oder, wenn sie zusammengenommen werden, $C_1$—$C_6$-Alkyliden, worin die Hydroxygruppen frei sind, oder $C_2$—$C_7$-Alkanoyl oder Benzoylester davon bedeuten.

2. Verbindung nach Anspruch 1, worin Z für —$CH_3$ steht.

3. Verbindung nach Anspruch 1, worin Z für —$CH_2SCH_3$ steht.

4. Verdindung nach Anspruch 1, worin Z für —$CH_2Hal$, worin Hal ein Halogenatom, vorzugsweise Chlor, bedeutet, steht.

5. Verdingung nach Anspruch 2, 3 oder 4, worin R die Formel (Ia)

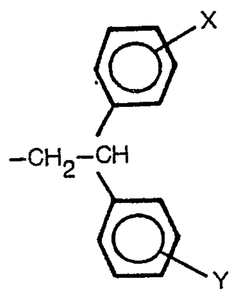

(Ia)

darstellt.

6. Verbindung nach Anspruch 2, 3 oder 4, worin R die Formel (Ib)

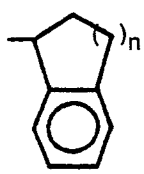

(Ib)

darstellt.

7. Verbindung nach Anspruch 2, 3 oder 4, worin R die Formel (Ic)

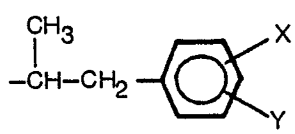

(Ic)

aufweist.

8. Verbindung nach Anspruch 2, 3 oder 4, worin R die Formel (Ie)

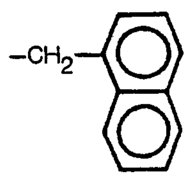

(Ie)

aufweist.

9. Verbindung nach Anspruch 2, 3 oder 4, worin R ein cycloalkylrest mit 3 bis 11 Ringgliedern, vorzugsweise mit 5 bis 7 Ringgliedern, ist.

10. Verbindung nach Anspruch 5, 6 oder 7, worin X und Y Wasserstoff bedeuten.

11. Verbindung nach Anspurch 10, worin Q für 5'-Desoxy-β-D-ribose steht, und $OR_2'$ und $OR_3'$ Hydroxy oder Acetylester davon sind.

12. Verbindung nach Anspruch 11, wenn auf die Ansprüche 2 und 5 rückbezogen, mit dem Namen: 5'Desoxy-$N^6$-(2,2-Di-phenyläthyl)-adenosin.

13. Verbindung nach Anspruch 11, wenn auf die Ansprüche 2 und 6 rückbezogen, welche ausgewählt ist aus: 5'-Desoxy-$N^6$-(1-indanyl)-andenosin; $N^6$-1-Tetralinyl-5'-desoxyadenosin; und $N^6$-1-Tetralinyl-5'-desoxyadenosin-2', 3'-di-O-acetyl.

14. Verbindung nach Anspruch 11, wenn auf die Ansprüche 2 und 7 rückbezogen, welche ausgewählt ist aus: (R)-5'-Desoxy-$N^6$-(1-methyl-2-phenyläthyl)-adenosin und (S)-5'-Desoxy-$N^6$-(1-methyl-2-phenyläthyl)-adenosin.

15. Verbindung nach Anspruch 8, wenn auf den Anspruch 2, rückbezogen, welche ausgewählt ist aus: $N^6$-α-Naphthylmethyl-5'-desoxyadenosin und $N^6$-α-Naphthylmethyl-3'desoxyadenosin-2',3'-di-O-acetyl.

16. Verbindung nach Anspruch 9, wenn auf den Anspruch 2 rückbezogen, worin Q 5'-Desoxy-β-D-ribose ist, und $OR_2'$ und $OR_3'$ jeweils Hydroxy oder Acetyloxy sind.

17. Verbindung nach Anspruch 16 mit dem Namen 5'-Desoxy-$N^6$-(cyclopentyl)-adenosin.

18. Verbindung nach Anspruch 10, wen auf den Anspruch 3 rückbezogen, worin $R_2'$ und $R_3'$ jeweils unabhängig voneinander Wasserstoff, Acetyl, Benzoyl oder, wenn sie zusammengenommen werden, Isopropyliden sind.

19. Verdindung nach Anspruch 18, wenn auf den Anspruch 5 rückbezogen, mit dem Namen: 5'-Desoxy-5'-methylthio-$N^6$-(2,2-diphenyläthyl)-adenosin.

20. Verbindung nach Anspruch 18, wenn weiters auf den Anspruch 6 rückbezogen, met dem Namen: 5'-Desoxy-5'methylthio-$N^6$-(1-indanyl)-adenosin.

21. Verbindung nach Anspruch 18, wenn weiters auf den Anspruch 7 rückbezogen, welche ausgewählt ist aus: (R)-5'-Desoxy-5'-methylthio-$N^6$-(1-methyl-2-phenyläthyl)-adenosin und (S)-5'-Desoxy-5'-methylthio-$N^6$-(1-methyl-2-phenyläthyl)-adenosin.

22. Verbindung nach Anspruch 9, wenn auf den Anspruch 3 rückbezogen, worin $R_2'$ und $R_3'$ jeweils unabhängig voneinander Wasserstoff, Acetyl, Benzoyl oder, wenn sei zusammengenommen werden, Isopropyliden sind.

23. Verbindung nach Anspruch 22, worin $R_2'$ und $R_3'$ Wasserstoff sind.

24. Verbindung nach Anspruch 23, welche 5'-Desoxy-5'-methylthio-$N^6$-(cyclopentyl)-adenosin ist.

25. Verbindung nach Anspruch 10, wenn auf den Anspruch 4, rückbezogen, worin $R_2'$ und $R_3'$ jeweils unabhängig voneinander Wasserstoff, Acetyl oder Benzoyl sind oder $R_2'$ und $R_3'$, wenn sie zusammengenommen werden, Isopropyliden sind.

26. Verbindung nach Anspruch 25, wenn weiters auf den Anspruch 5 rückbezogen, welche 5'-Desoxy-5'-chlor-$N^6$-(2,2-diphenyläthyl)-adenosin ist.

27. Verbindung nach Anspruch 25, wenn weiters auf den Anspruch 6 rückbezogen, welche ausgewählt ist aus: 5'-Desoxy-5'-chlor-$N^6$-(1-indanyl)-adenosin und (R)-$N^6$-(1-Indanyl)-5'-desoxy-5'-chlor-adenosin.

28. Verbindung nach Anspruch 25, wenn weiters auf den Anspruch 7 rückbezogen, welche ausgewählt ist aus: (R)-5'-Desoxy-5'-chlor-$N^6$-(1-methyl-2-phenyläthyl)-adenosin und (S)-5'-Desoxy-5'-chlor-$N^6$-(1-methyl-2-phenyläthyl)-adenosin.

29. Verbindung nach Anspruch 9, wenn auf den Anspruch 4 rückbezogen, worin $R_2'$ und $R_3'$ jeweils unabhängig voneindander Wasserstoff, Acetyl, Benzoyl oder, wenn ist sie zusammengenommen werden, ein Isopropyliden sind.

30. Verbindung nach Anspruch 29, worin $R_2'$ und $R_3'$ jeweils Wasserstoff sind.

31. Verbindung nach Anspruch 30, welche 5'-Desoxy-5'-chlor-$N^6$-(cyclopentyl)-adenosin ist.

32. Pharmazeutische Zusammensetzung, welche eine therapeutisch wirksame Minge einer wie in irgendeinem vorhergehenden Anspruch beanspruchten Verbindung zusammen mit einem pharmazeutisch akzeptablen Träger oder Verdünnungsmittel umfaßt.

33. Verbindung wie in irgendeinem der Asprüche 1 bis 31 beansprucht, vorzugsweise in Dosiseinheitsform, zur Verwendung in einer Methode der Benhandlung von Psychosen bei einem daran leidenden Säuger.

34. Verbindung wei in irgendeinem der Asprüche 1 bis 31 beansprucht, vorzugsweise in Dosiseinheitsform, zur Verwendung in einer Methode der Buhandlung von Hypertonie bei einem daran leidenden Säuger.

35. Verbindung wei in irgendeinem der Asprüche 1 bis 31 beansprucht, vorzungsweise in Dosiseinheitsform, zur Verwendung in einer Methode der Schmerzbehandlung bei einem daran leidenden Säuger.

36. Verfahren zur Herstellung einer Verbindung wie im Anspruch 1 beansprucht, welches die Umsetzung eines N-6-substituierten Purins der Formel

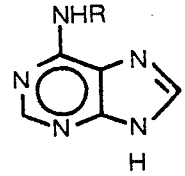

worin R wie im Anspruch 1 definiert ist, mit einer Verbindung der Formel

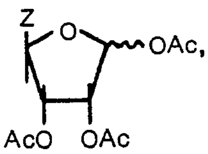

worin z wie im Anspruch 1 definiert ist, bei erhöhten Temperaturen während etwa einer bis etwa zwölf Stunden und gewünschtenfalls Überführung der resultierenden freien Base in ein pharmazeutisch akzeptables Saüreadditionssalz davon umfaβt.

**Patentansprüche für den Vertragsstaat: AT**

1. Verfahren zur Herstellung einer Verbindung der folgenden allgemeinen Formel (I)

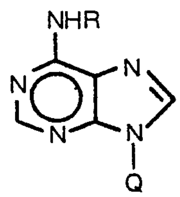

(I)

oder ein pharmazeutisch akzeptablen Säureadditionssalzes davon, worin

R Cycloalkyl mit 3 bits II Ringgliedern ist oder R einr der folgenden Gruppen der Formeln

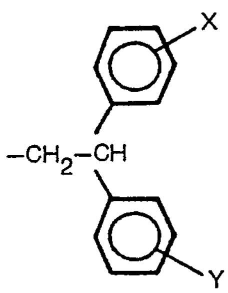 ,

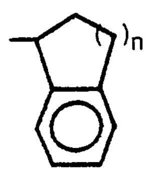

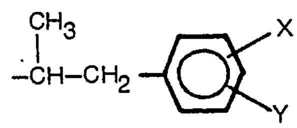 ,

oder

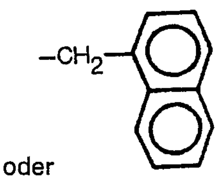

(Ia) (Ib) (Ic) (Ie)

worin

n für 1, 2 oder 3 steht;

X und Y jeweils unabhängig voneinander H, $C_1$—$C_6$-Alkyl, Hydroxy, $C_1$—$C_6$-Alkoxy, Benzyloxy, Nitro, Amino oder Halogen bedeuten, ist, und

Q die Formel

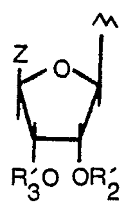

darstellt, worin

Z für $CH_3$, $CH_2Hal$, worin Hal ein Halogenatom oder $CH_2SCH_3$ bedeutet, steht; und

$R_2'$ oder $R_3'$ jeweils unabhängig voneinander H, $C_1$—$C_6$-Alkyl, $C_2$—$C_7$-Alkanoyl, Benzoyl, Benzoyl substituiert durch $C_1$—$C_6$-Alkyl, $C_1$—$C_6$-Alkoxy oder Halogen bedeuten oder, wenn sie zusammengenommen werden, $C_1$—$C_6$-Alkyliden, worin die Hydroxygruppen frei sind, oder $C_2$—$C_7$-Alkanoyl oder Benzoylester davon bedeuten, welches Verfahren die Umsetzung eines N-6-substituierten Purins der Formel

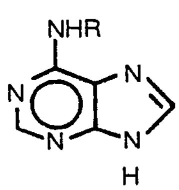

worin R wie oben definiert ist, mit einer Verbindung der Formel

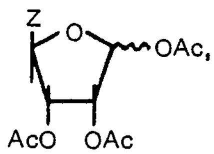

worin Z wie oben definiert ist, bei erhöhten Temperaturen während etwa 1 bis etwa 12 Stunden und gewünschtenfalls Überführung der restultierenden freien Base in ein pharmazeutisch akzeptables Säureadditionssalz davon umfaßt.

2. Verfahren nach Anspruch 1, worin für $—CH_3$ steht.

3. Verfahren nach Anspruch 1, worin Z für $—CH_2SCH_3$ steht.

4. Verfahren nach Anspruch 1, worin Z für $—CH_2Hal$, worin Hal ein Halogenatom, vorzugsweise Chlor, bedeutet, steht.

5. Verfahren nach Anspruch 2, 3 oder 4, worin R die Formel (Ia)

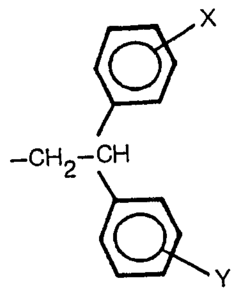

(Ia)

darstellt.

6. Verfahren nach Anspruch 2, 3 oder 4, worin R die Formel (Ib)

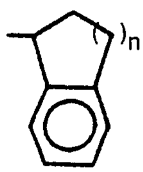

(Ib)

darstellt.

7. Verfahren nach Anspruch 2, 3 oder 4, worin R die Formel (Ic)

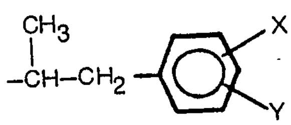

(Ic)

darstellt.

8. Verfahren nach Anspruch 2, 3 oder 4, worin R die Formel (Ie)

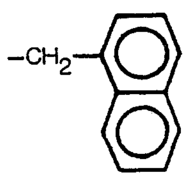

(Ie)

darstellt.

9. Verfahren nach Anspruch 2, 3 oder 4, worin R ein cycloalkylrest mit 3 bis ll Ringgliedern, vorzugsweise mit 5 bis 7 Ringgliedern, darstellt.

10. Verfahren nach Anspruch 5, 6 oder 7, worin X und Y Wasserstoff darstellen.

11. Verfahren nach Anspurch 10, worin Q 5'-Desoxy-β-D-ribose bedeutet, und $OR_2'$ und $OR_3'$ Hydroxy oder Acetylester davon sind.

12. Verfahren nach Anspruch 11, wenn auf die Ansprüche 2 und 5 rückbezogen, in welchem eine Verbindung mit dem folgenden Namen hergestellt wird: 5'Desoxy-$N^6$-(2,2-diphenyläthyl)-adenosin.

13. Verfahren nach Anspruch 11, wenn auf die Ansprüche 2 und 6 rückbezogen, welchem eine Verbingdung mit einer der folgenden Namen hergestellt wird: 5'-Desoxy-$N^6$-(1-indanyl)-andenosin, $N^6$-1-Tetralinyl-5'-desoxyadenosin; und $N^6$-1-Tetralinyl-5'-desoxyadenosin-2', 3'-di-O-acetyl.

14. Verfarhren nach Anspruch 11, wenn auf die Ansprüche 2 und 7 rückbezogen, in welchem eine Verbindung mit einem der folgenden Namen herestellt wird: (R)-5'-Desoxy-$N^6$-(1-methyl-2-phenyläthyl)-adenosin und (S)-5'-Desoxy-$N^6$-(1-methyl-2-phenyläthyl)-adenosin.

15. Verfahren nach Anspruch 8, wenn auf den Anspruch 2, rückbezogen, in welchem eine Verbindung mit einem der folgenden Namen hergestellt wird: $N^6$-α-Naphthylmethyl-5'-desoxyadenosin und $N^6$-α-Naphthylmethyl-3'desoxyadenosin-2',3'-di-O-acetyl.

16. Verfahren nach Anspruch 9, wenn auf den Anspruch 2 rückbezogen, in welchem Q 5'-Desoxy-β-D-ribose ist, und $OR_2'$ und $OR_3'$ jeweils Hydroxy oder Acetyloxy darstellen.

17. Verfahren nach Anspruch 16 in welchen die folgende Verbindung hergestellt wird: 5'-Desoxy-$N^6$-(cyclopentyl)-adenosin.

18. Verfahren nach Anspruch 10, wenn auf den Anspruch 3 rückbezogen, worin $R_2'$ und $R_3'$ jeweils unabhängig voneinander Wasserstoff, Acetyl, Benzoyl oder, wenn sie zusammengenommen werden, Isopropyliden bedeuten.

19. Verfahren nach Anspruch 18, wenn weiters auf den Anspruch 5 rückbezogen, in welchem die folgende Verbindung hergestellt wird: 5'-Desoxy-5'-methylthio-$N^6$-(2,2-diphenyläthyl)-adenosin.

20. Verfahren nach Anspruch 18, wenn weiters auf den Anspruch 6 rückbezogen, in welchem die folgende Verbindung hergestellt wird: 5'-Desoxy-5'methylthio-$N^6$-(1-indanyl)-adenosin.

21. Verfahren nach Anspruch 18, wenn weiters auf den Anspruch 7 rückbezogen, in welchem eine der folgenden Verbindungen hergestellt wird: (R)-5'-Desoxy-5'-methylthio-$N^6$-(1-methyl-2-phenyläthyl)-adenosin und (S)-5'-Desoxy-5'-methylthio-$N^6$-(1-methyl-2-phenyläthyl)-adenosin.

22. Verfahren nach Anspruch 9, wenn weiters auf den Anspruch 3 rückbezogen, worin $R_2'$ und $R_3'$ jeweils unabhängig voneinander Wasserstoff, Acetyl, Benzoyl oder, wenn sie zusammengenommen werden, Isopropyliden darstellen.

23. Verfahren nach Anspruch 22, worin $R_2'$ und $R_3'$ Wasserstoff bedeuten.

24. Verfahren nach Anspruch 23, in welchem eine Verbindung der folgenden Formel hergestellt wird: 5'-Desoxy-5'-methylthio-$N^6$-(cyclopentyl)-adenosin.

25. Verfahren nach Anspruch 10, wenn weiters auf den Anspruch 4, rückbezogen, worin $R_2'$ und $R_3'$ jeweils unabhängig voneinander Wasserstoff, Acetyl oder Benzoyl darstellen oder $R_2'$ und $R_3'$, wenn sie zusammengenommen werden, Isopropyliden darstellen.

26. Verfahren nach Anspruch 25, wenn weiters auf den Anspruch 5 rückbezogen, in welchem eine Verbindung der folgenden Formel hergestellt wird: 5'-Desoxy-5'-chlor-$N^6$-(2,2-diphenyläthyl)-adenosin.

27. Verfahren nach Anspruch 25, wenn weiters auf den Anspruch 6 rückbezogen, in welchem eine der folgenden Verbindungen hergestellt wird: 5'-Desoxy-5'-chlor-$N^6$-(1-indanyl)-adenosin und (R)-N-6-(1-Indanyl)-5'-desoxy-5'-chlor-adenosin.

28. Verfahren nach Anspruch 25, wenn weiters auf den Anspruch 7 rückbezogen, in welchem einer der folgenden Verbindungen hergestellt wird: (R)-5'-Desoxy-5'-chlor-$N^6$-(1-methyl-2-phenyläthyl)-adenosin und (S)-5'-Desoxy-5'-chlor-$N^6$-(1-methyl-2-phenyläthyl)-adenosin.

29. Verfahren nach Anspruch 9, wenn auf den Anspruch 4 rückbezogen, in welchem $R_2'$ und $R_3'$ jeweils unabhängig voneindander Wasserstoff, Acetyl, Benzoyl oder, wenn sie zusammengenommen werden, ein Isopropyliden darstellen.

30. Verfahren nach Anspruch 29, worin $R_2'$ und $R_3'$ jeweils Wasserstoff sind.

31. Verfahren nach Anspruch 30, in welchem die folgende Verbindung hergestelle wird: 5'-Desoxy-5'-chlor-$N^6$-(cyclopentyl)-adenosin.

32. Verfahren zur Herstellung einer pharmazeutischen Zusammensetzung, welche die Kombination einer therapeutisch wirksamen Menge einer Verbindung, welche mittels eines wie in irgendeinem vorhergehenden Anspruch beanspruchten Verfahrens hergestellt wurde, zusammen mit einem pharmazeutisch akzeptablen Träger oder Verdünnungsmittel umfaßt.

33. Verbindung, welche mittels eines wie in irgendeinem der Asprüche 1 bis 31 beanspruchten Verfahrens hergestellt wurde, vorzugsweise in Dosiseinheitsform, zur Verwendung in einer Methode zur Benhandlung von Psychosen bei einem daran leidenden Säuger.

34. Verbindung, welche mittels eines wie in irgendeinem der Asprüche 1 bis 31 beanspruchten Verfahrens hergestellt wurde, vorzugsweise in Dosiseinheitsform, zur Verwendung in einer Methode zur Buhandlung von Hypertonie bei einem daran leidenden Säuger.

35. Verbindung, welche mittels eines wie in irgendeinem der Asprüche 1 bis 31 beanspruchten Verfahrens hergestellt wurde, vorzungsweise in Dosiseinheitsform, zur Verwendung in einer Methode zur Schmerzbehandlung bei einem daran leidenden Säuger.

**Revendications pour les Etats contractants: BE CH DE FR GB IT LI LU NL SE**

1. Un dérivé de formule générale suivante:

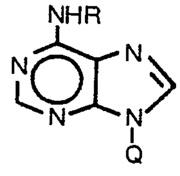

ou un de ses sels d'addition d'acides pharmaceutiquement acceptables, dans laquelle:

R représente un radical cyclolkyle renfermant de 3 à II éléments dans le cycle, ou R représente l'un des groupes ayant les formules suivantes:

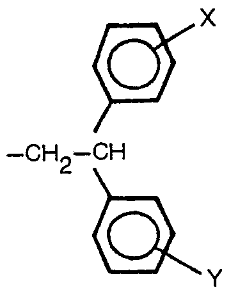 , 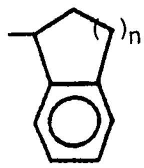 , 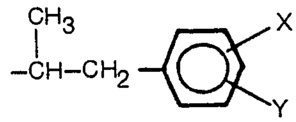 ou 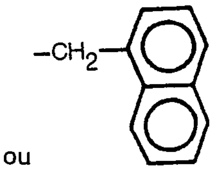

dans lesquelles:

n est égal à un, deux ou trois;

X et Y représentent chacun, indépendamment l'un de l'autre, un atome d'hydrogéne, un radical alkyle en $C_1$ à $C_6$, un groupe hydroxy, un radical alkoxy en $C_1$ à $C_6$, benzyloxy, un groupe nitro, amino ou un atome d'halogène; et

Q répond à la formule:

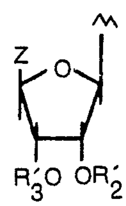

dans laquelle:

Z représente $CH_3$, $CH_2Hal$ dans lequel Hal représente un atome d'halogène, ou $CH_2SCH_3$; et

$R_2'$ ou $R_3'$ représentent chacun, indépendamment l'un de l'autre, un atome d'hydrogène, un radical alkyle en $C_1$ à $C_6$, alkanoyle en $C_2$ à $C_7$, benzoyle, benzoyle substitué par un radical alkyle en $C_1$ à $C_6$ alkoxy en $C_1$ à $C_6$ ou un atome d'halogène ou, lorsqu'ils sont pris ensemble, ils représentent un radical alkylidène en $C_1$ à $C_6$ dans lequel les groupes hydroxy sont libres ou un radical alkanoyle en $C_2$ à $C_6$ ou leurs esters de benzoyle.

2. Un dérivé selon la revendication 1, dans lequel Z représente —$CH_3$.

3. Un dérivé selon la revendication 1, dans lequel Z représente —$CH_2SCH_3$.

4. Un dérivé selon la revendication 1, dans lequel Z représente —$CH_2Hal$, avec Hal représentant un atome d'halogène, de préférence de chlore.

5. Un dérivé selon la revendication 2, 3 ou 4, dans lequel R répond à la formule (Ia):

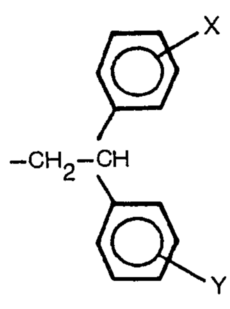

(Ia)

6. Un dérivé selon la revendication 2, 3 ou 4, dans lequel R répond à la formule (Ib):

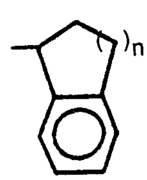

(Ib)

7. Un dérivé selon la revendication 2, 3 ou 4, dans lequel R répond à la formule (Ic):

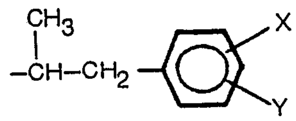

(Ic)

8. Un dérivé selon la revendication 2, 3 ou 4, dans lequel R répond à la formule (Ie):

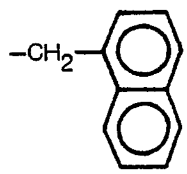

(Ie)

9. Un dérivé selon la revendication 2, 3 ou 6, dans lequel R représente un radical cycloalkyle renfermant de 3 à 11 éléments dans le cycle, de préférence de 5 à 7 éléments dans le cycle.

10. Un dérivé selon la revendication 5, 6 ou 7, dans lequel X et Y sont des atomes d'hydrogène.

11. Un dérivé selon la revendication 10, dans lequel Q représente le 5'-déoxy-β-D-ribose et $OR_2'$ et $OR_3'$ représentent un groupe hydroxy ou leurs esters acétyliques.

12. Un dérivé selon la revendication 11, pris en liaison avec les revendications 2 et 5, et consistant en la 5'-déoxy-$N^6$-(2,2-diphényléthyl)adénosine.

13. Un dérivé selon la revendication 11, pris en liaison avec les revendications 2 et 6, et choisi parmi: la 5'-déoxy-$N^6$-(1-indanyl)adénosine, la $N^6$-1-tétralinyl-5'-déoxyadénosine; et le $N^6$-1-tétralinyl-5'-déoxyadénosine-2',3'-di-O-acétyle.

14. Un dérivé selon la revendication 11, pris en liaison avec les revendications 2 et 7, et choisi parmi: la (R)-5'déoxy-$N^6$-(1-méthyl-2-phényléthyl)adénosine; et la (S)-5'déoxy-$N^6$-(1-méthyl-2-phényléthyl)-adénosine.

15. Un dérivé selon la revendication 8, pris en liaison avec la revendication 2, et choisi parmi: la $N^6$-α-naphtylméthyl-5'-déoxyadénosine; et la $N^6$-α-naphtylméthyl-3'-déoxyadénosine-2',3'-di-O-acétyle.

16. Un dérivé selon la revendication 9, pris en liaison avec la revendication 2, dans lequal Q consiste en le 5'-déoxy-β-D-ribose et $OR_2'$ et $OR_3'$ représentent chacun un groupe hydroxy ou un radical acétyloxy.

17. Un dérivé selon la revendication 16, et consistant notamment en 5'-déoxy-$N_6$-(cyclopentyl)adénosine.

18. Un dérivé selon la revendication 10, pris en liaison evec la revendicatio 3, dans lequel $R_2'$ et $R_3'$ représentent chacun, indépendamment l'un de l'autre, un atome d'hydrogène, forment un radical acétyle, benzoyle ou, lorsqu'ils sont pris ensemble , un radical isopropylidéne.

19. Un dérivé selon la revendication 18, pris en outre en liaison avec la revendication 5, et consistant notamment en la 5'-déoxy-5'-méthylthio-$N^6$-(2,2-diphényléthyl)adénosine.

20. Un dérivé selon la revendication 18, pris en outre en liaison avec la revendication 6, et consistant notamment en la 5'-déoxy-5'-méthylthio-$N^6$-(1-indanyl)adénosine.

21. Un dérivé selon la revendication 18, pris en outre en liaison avec le revendiation 7, et consistant notamment en la (R)-5'-déoxy-5'-methylthio-$N^6$-(1-méthyl-2-phényléthyl)-adénosine; et la (S)-5'-déoxy-5'-methylthio-$N^6$-(1-méthyl-2-phényléthyl)-adénosine.

22. Un dérivé selon la revendication 9, pris en liaison avec la revendication 3, dans lequel $R_2'$ et $R_3'$ représentent chacun, indépendamment l'un de l'autre, un atome, d'hydrogène, un radical acétyle, benzoyle ou, lorsqu'ils sont pris ensemble, forment un radical isopropylidéne.

23. Un dérivé selon la revendication 22, dans lequel $R_2'$ et $R_3'$ sont des atomes d'hydrogène.

24. Un dérivé selon la revendication 23, et consistant notamment en la 5'-déoxy-5'-methylthio-$N^6$-(cyclopentyl)-adénosine.

25. Un dérivé selon la revendication 10, pris en liaison avec la revendication 4, dans lequel $R_2'$ et $R_3'$ représentent chacun, indépendamment l'un de l'autre, un atome d'hydrogène, un radical acétyle ou benzoyle, ou $R_2'$ et $R_3'$, lorsqu'ils sont pris ensemble, forment en radical isopropylidène.

26. Un dérivé selon la revendication 25, pris en outre en liaison avec la revendication 5, et consistant notamment en: 5'-déoxy-5'-chloro-$N^6$-(2,2-diphényléthyl)-adénosine.

27. Un dérivé selon la revendication 25, pris en outre en liaison avec la revendication 6, et consistant notamment en: la 5'-déoxy-5'-chloro-$N^6$-(1-indanyl)adénosine; et la (R)-N-6-(1-indanyl)-5'-déoxy-5'-chloroadénosine.

28. Un dérivé selon la revendication 25, pris en outre en liaison avec la revendiation 7, en choisi parmi: la (R)-déoxy-5'-chloro-$N^6$-(1-(méthyl-2-phényléthyl)adénosine; et la (S)-5'-déoxy-5'-chloro-$N^6$-(1-(méthyl-2-phényléthyl)adénosine.

29. Un dérivé selon la revendication 9, pris en liaison avec la revendication 4, dans lequel $R_2'$ et $R_3'$ représentent chacun, indépendamment l'un de l'autre, un atome d'hydrogène, un radical acétyle, benzoyle ou, lorsqu'ils, sont pris ensemble, forment un radical isopropylidène.

30. Un dérivé selon la revendication 29, dans lequel $R_2'$ et $R_3'$ sont des atomes d'hydrogéne.

31. Un dérivé selon la revendication 30, et notamment la 5'-déoxy-5'-chloro-$N^6$-(cyclopentyl)adénosine.

32. Une composition pharmaceutique comprenant une quantité thérapeutiquement efficace d'un dérivé selon l'une quelconque des revendications précédentes, associé à un support ou diluant pharmaceutiqument acceptable.

33. Pour l'utilisation dans un procédé de traitement de psychoses chez un mammifère qui en souffre, un dérivé selon l'une quelconque des revendications 1 à 31, de préference sous forme de dosage unitaire.

34. Pour l'utilisation dans un procédé de traitement de l'hypertension chez un mammifère qui en souffre, un dérivé selon l'une quelconque des revendications 1 à 31, de préférence sous forme de dosage unitaire.

35. Pour l'utilisation dans une méthode de traitement de le douleur chez un mamifère qui en souffre, un dérivé selon l'une quelconque des revendications 1 à 31, de préference sous forme de dosage unitaire.

36. Un procede de préparation d'un dérivé selon la revendication 1, qui consiste à faire réagir une purine substituée en positions N-6, de formule:

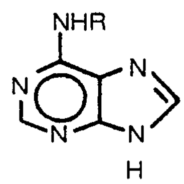

dans laquelle R est tel que défini dans la revendication 1, avec un dérivé de formule:

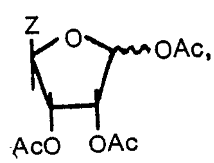

dans laquelle Z est tel que défini dans la revendication 1, à des températures élevées, pendant une durée d'environ 1 à 12 heures et, si on le souhaite, à convertir la base libre obtenue en un de ses sels d'addition d'acides pharmaceutiquement acceptables.

**Revendications pour l'Etat contractant: AT**

1. Un procédé de préparation d'un dérivé de formule générale suivante (I)

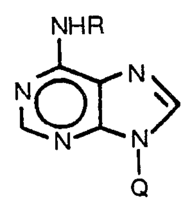

(I)

ou un de ses sels d'addition d'acides pharmaceutiquement acceptables,
dans laquelle:

R représente un radical cycloalkyle renfermant de 3 à 11 éléments dans le cycle, ou R représente l'un des groupes ayant les formules suivantes:

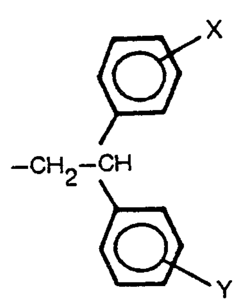 ,

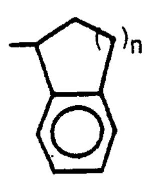

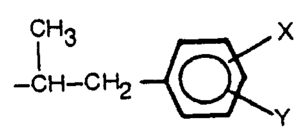 ,

ou

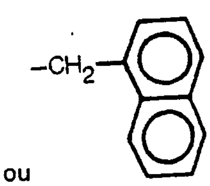

dans lesquelles:

n est égal à un, deux ou trois;

X et Y représentent chacun, indépendamment l'un de l'autre, un atome d'hydrogène, un radical alkyle en $C_1$ à $C_6$, un groupe hydroxy, un radical alkoxy en $C_1$ à $C_6$, benzyloxy, un groupe nitro, amino ou un atome d'halogène; et

Q répond à la formule:

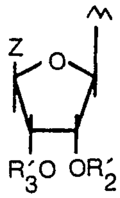

dans laquelle:

Z représente $CH_3$, $CH_2Hal$ dans lequel Hal représente un atome d'halogène ou $CH_2SCH_3$; et

$R_2'$ ou $R_3'$ représentent chacun, indépendamment l'un de l'autre un atome d'hydrogène, un radical alkyle en $C_1$ à $C_6$, alkanoyle en $C_2$ à $C_7$, benzoyle, benzoyle substitué par un radical alkyle en $C_1$ à $C_6$, alkoxy en $C_1$ à $C_6$ ou un atome d'halogène ou, lorsqu'ils sont pris ensemble, ils représentent un radical alkylidène en $C_1$ à $C_6$ dans lequel les groupes hydroxy sont libres ou un radical alkanoyle en $C_2$ à $C_7$ ou leurs esters de benzoyle,

ce procédé consistant à faire réagir une purine substituée en position N—6, de formule:

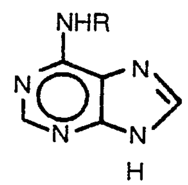

dans laquelle R est tel que défini ci-dessus
avec un dérivé de formule:

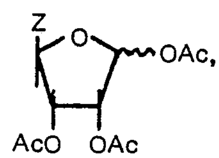

dans laquelle Z est tel que défini ci-dessus,
à des températures élevées, pendant une durée d'environ 1 à environ 12 heures et, si on le souhaite, à convertir la base libre obtenue en un de ses sels d'addition d'acides pharmaceutiquement acceptables.

2. Un procédé selon la revendication 1, dans lequel Z représente $—CH_3$.

3. Un procédé selon la revendication 1, dans lequel Z représente $—CH_2SCH_3$.

4. Un procédé' selon la revendication 1, dans lequel Z représente $—CH_2Hal$, avec Hal représentant un atome d'halogène, de préférence de chlore.

5. Un procédé selon la revendication 2, 3 ou 4, dans lequel R répond à la formule (Ia):

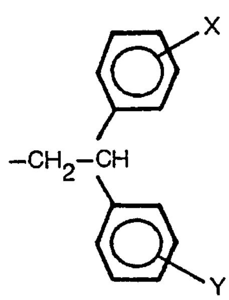

(Ia)

6. Un procédé selon la revendication 2, 3 ou 4, dans lequel R répond à la formule (Ib):

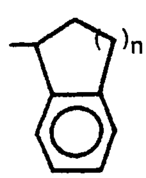

(Ib)

7. Un procédé selon la revendication 2, 3 ou 4, dans lequel R répond à la formule (Ic):

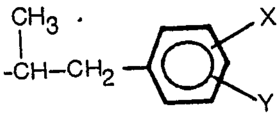

(Ic)

8. Un procédé selon la revendication 2, 3 ou 4, dans lequel R répond à la formule (Ie):

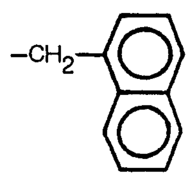

(Ie)

9. Un procédé selon la revendication 2, 3 ou 4, dans lequel R représente un radical cycloalkyle renfermant de 3 à 11 éléments dans le cycle, de préférence de 5 à 7 éléments dans le cycle.

10. Un procédé selon la revendication 5, 6 ou 7, dans lequel X et Y sont des atomes d'hydrogène.

11. Un procédé selon la revendication 10, dans lequel Q représente le 5'-déoxy-β-D-ribose et $OR_2'$ et $OR_3'$ représentent un groupe hydroxy ou leurs esters acétyliques.

12. Un procédé selon la revendication 11, pris en liaison avec les revendications 2 et 5, dans lequel on prépare un dérivé portant le nom suivant: 5'-déoxy-$N^6$-(2,2-diphényléthyl)adénosine.

13. Un procédé selon la revendication 11, pris en liaison avec les revendications 2 et 6, dans lequel on prépare un dérivé portant l'un des noms suivants: la 5'-déoxy-$N^6$-(1-indanyl)adénosine, la $N^6$-1-tétralinyl-5'-déoxyadénosine; et le $N^6$-1-tétralinyl-5'-déoxyadénosine-2',3'-di-O-acétyle.

14. Un procédé selon la revendication 11, pris en liaison avec les revendictions 2 et 7, dans lequel on prépare un dérivé portant l'un des noms suivants: la (R)-5'-déoxy-$N^6$-(1-méthyl-2-phényléthyl)adénosine; et la (S)-5'-déoxy-$N^6$-(1-méthyl-2-phényléthyl)adénosine.

15. Un procédé selon la revendication 8, pris en liaison avec la revendication 2, dans lequel on prépare un dérivé portant l'un des noms suivants: la $N^6$-α-naphtylméthyl-5'-déoxyadénosine; et le $N^6$-α-naphtylméthyl-3'-déoxyadénosine-2',3'-di-O-acétyle.

16. Un procédé selon la revendication 9, pris en liaison avec la revendication 2, dans lequel Q consiste en le 5'-déoxy-β-D-ribose et $OR_2'$ et $OR_3'$ représentent chacun un groupe hydroxy ou un radical acétyloxy.

17. Un procédé selon la revendication 16, dans lequel on prépare le dérivé suivant: la 5'-déoxy-$N^6$-(cyclopentyl)adénosine.

18. Un procédé selon la revendication 10, pris en liaison avec la revendication 3, dans lequel $R_2'$ et $R_3'$ représentent chacun, indépendamment l'un de l'autre, un atome d'hydrogène, un radical acétyle, benzoyle, ou, lorsqu'ils sont pris ensemble, forment un radical isopropyliène.

19. Un procédé selon la revendication 18, pris en outre en liaison avec la revendication 5, dans lequel on prépare le dérivé suivant: la 5'-déoxy-5'-méthylthio-$N^6$-(2,2-diphényléthyl)adénosine.

20. Un procédé selon la revendication 18, pris en outre en liaison avec la revendication 6, dans lequel on prépare le dérive suivant: la 5'-déoxy-5'-méthylthio-$N^6$-(1-indanyl)adénosine.

21. Un procédé selon la revendication 18, pris en outre en liaison ave la revendication 7, dans lequel on prépare l'un des dérivés suivants: la (R)-5'-déoxy-5'-méthylthio-$N^6$-(1-méthyl-2-phényléthyl)adénosine; et la (S)-5'-déoxy-5'-méthylthio-$N^6$-(1-méthyl-2-phényléthyl)adénosine.

22. Un procédé selon la revendication 9, pris en liaison avec la revendication 3, dans lequel $R_2'$ et $R_3'$ représentent chacun, indépendamment l'un de l'autre, un atome d'hydrogène, un radical acétyle, benzoyle ou, lorsqu'ils sont pris ensemble, forment un radical isopropylidène.

23. Un procédé selon la revendication 22, dans lequel $R_2'$ et $R_3'$ sont des atomes d'hydrogène.

24. Un procédé selon la revendication 23, dans lequel on prépare un dérivé répondant à la formule suivante: la 5'-déoxy-5'-méthylthio-$N^6$-(cyclopentyl)adénosine.

25. Un procédé selon la revendication 10, pris en liaison avec la revendication 4, dans lequel $R_2'$ et $R_3'$ représentent chacun, indépendamment l'un de l'autre, un atome d'hydrogène, un radical acétyle ou benzoyle, ou $R_2'$ et $R_3'$, lorsqu'ils sont pris ensemble, représentent un radical isopropylidène.

26. Un procédé selon la revendication 25, pris en outre en liaison avec la revendication 5, dans lequel on prépare un dérivé répondant à la formule suivante: la 5'-déoxy-5'-chloro-$N^6$-(2,2-diphényléthyl)-adénosine.

27. Un procédé selon la revendication 25, pris en outre en liaison avec la revendication 6, dans lequel on préparé l'un des dérivés suivants: la 5'-déoxy-5'-chloro-$N^6$-(1-indanyl)adénosine; et la (R)-N-6-(1-indanyl)-5'-déoxy-5'-chloroadénosine.

28. Un procédé selon la revendication 25, pris en outre en liaison avec la revendication 7, dans lequel on prépare l'un des dérivés suivants: la (R)-5'-déoxy-5'-chloro-$N^6$-(1-méthyl-2-phényléthyl)adénosine; et la (S)-5'-déoxy-5'-chloro-$N^6$-(1-méthyl-2-phényléthyl)adénosine.

29. Un procédé selon la revendication 9, pris en liaison avec la revendication 4, dans lequel $R_2'$ et $R_3'$

représentent chacun, indépendamment l'un de l'autre, un atome d'hydrogène, un radical acétyle, benzoyle, ou, lorsqu'il sont pris ensemble, forment un radical isopropylidène.

30. Un procédé selon la revendication 29, dans lequel $R_2'$ et $R_3'$ sont des atomes d'hydrogène.

31. Un procédé selon la revendication 30, dans lequel on prépare le dérivé suivant: la 5'-déoxy-5'-chloro-$N^6$-(cyclopentyl)adénosine.

32. Un procédé de préparation d'une composition pharmaceutique consistant à associier une quantité thérapeutiquement efficace d'un dérivé préparé par un procédé selon l'une quelconque des revendications précédentes, associé à un support ou diluant pharmaceutiquement acceptable.

3. Pour l'utilisation dans un procédé de traitement des psychoses chez un mammifère qui un souffre, un dérivé préparé par un procédé selon l'une quelconque des revendications 1 à 31, de préférence sous forme de dosage unitaure.

34. Pour l'utilisation dans un procédé de traitement de l'hypertension chez un mammifère qui en souffre, un dérivé préparé par un procédé selon l'une quelconque des revendications 1 à 31, de préférence sous forme de dosage unitaire.

35. Pour l'utilisation dans une méthode de traitement de la douleur chez un mammifère qui en souffre, un dérivé préparé par un procédé selon l'une quelconque des revendications 1 à 31, de préférence sous forme de dosage unitaire.